# EUROPÄISCHE PATENTSCHRIFT

(11) **EP 2 241 403 B1**
(45) Veröffentlichungstag und Bekanntmachung des Hinweises auf die Patenterteilung: **27.02.2019**
(21) Anmeldenummer: 10002411.6
(22) Anmeldetag: 09.03.2010
(51) Int. Cl.: B25B 7/02, B25B 9/00, B25J 15/10, A61B 17/29, A61B 17/02, A61B 17/30, A61B 10/06, A61B 17/22, A61B 17/34, A61B 17/221, A61B 17/00

(54) **Manipulatorwerkzeug und Halte- und/oder Spreizwerkzeug mit wenigstens einem Manipulatorwerkzeug**
Manipulator tool and holding and/or widening tool with at least one manipulator tool
Outil de manipulation et outil pour fixer et/ou écarter avec au moins un outil de manipulation

(30) Priorität: 19.04.2009 DE 102009017591
(43) Veröffentlichungstag der Anmeldung: 20.10.2010
(73) Patentinhaber: Bannasch, Rudolf, 13053 Berlin (DE); Kniese, Leif, 14129 Berlin (DE); Karl Storz SE & Co. KG, 78532 Tuttlingen (DE)
(72) Erfinder: Bannasch, Rudolf Dr, 13053 Berlin (DE); Kniese, Leif, 14129 Berlin (DE)
(74) Vertreter: Grünecker Patent- und Rechtsanwälte PartG mbB

(56) Entgegenhaltungen:
- WO-A2-2009/039231
- DE-A1-102005 010 380
- DE-A1-102007 026 721
- DE-B3-102006 009 559
- DE-U1- 20 318 845
- FR-A- 545 837
- US-A- 4 719 826
- US-A1- 2004 183 348

## Beschreibung

Die Erfindung betrifft ein Halte- und/oder Spreizwerkzeug mit wenigstens zwei bezüglich eines Zwischenraumes aneinander gegenüberliegenden Backen, von denen wenigstens eine Backe bezüglich der anderen Backe beweglich ist.

Ein biegsames Manipulatorwerkzeug mit einem gegenüber einem proximalen Ende in wenigstens einer Manipulationsebene beweglichen distalen Ende, mit wenigstens zwei nebeneinander beabstandet verlaufenden, wenigstens in der Manipulationsebene flexiblen und sich vom proximalen Ende zum distalen Ende erstreckenden Wangen, die zwischen dem proximalen Ende und dem distalen Ende durch wenigstens ein wenigstens zugfestes Scharnierelement gegeneinander scherbeweglich verbunden und am proximalen Ende voneinander beabstandet gehalten sind, wobei die eine Wange wenigstens biegesteif und die andere Wange wenigstens zugfest ausgestaltet und die wenigstens zugfeste Wange an ihrem distalen Ende mit der wenigstens biegesteifen Wange Zugkraft übertragend verbunden ist, ist beispielsweise aus der DE-A-199164111 sowie der EP-A-1040999 und der EP-A-1316651 bekannt. Am proximalen Ende sind die Wangen unverschieblich befestigt, das distale Ende ist aufgrund der Struktur der Vorrichtung gegenüber dem proximalen Ende beweglich, wenn von außen eine Kraft auf eine der Wangen wirkt. Der Vorteil der in diesen Druckschriften beschriebenen Vorrichtung liegt darin, dass unter Wirkung der von einem Körper auf eine der Wangen ausgeübten Kraft sich die Geometrie so ändert, dass die Vorrichtung den Körper zu umgreifen versucht. Dies wird durch die Führung der beiden Wangen mittels der Scharnierelemente erreicht, so dass sich die unbelasteten Bereiche der Vorrichtung entgegen der Kraft relativ zum Angriffspunkt der Kraft hin bewegen.

Das wenigstens eine Scharnierelement und die wenigstens eine Wange sind wenigstens zugfest, können also zumindest Zugkräfte übertragen. In der einfachsten Form können daher als Scharnierelemente und als eine Wange biegeschlaffe Zugmittel wie Seile, Bänder oder Riemen verwendet werden. Mit dem Ausdruck "wenigstens zugfest" soll zum Ausdruck gebracht werden, dass diese Elemente über die reine Zugfestigkeit hinaus als Mindestanforderung biegesteif sein können, also auch Druckkräfte übertragen können.

Die biegesteife Wange ist flexibel, aber nicht biegeschlaff, vorzugsweise elastisch und kann Druckkraft aufnehmen. Sie kann zusätzlich zugfest ausgestaltet sein.

Das wenigstens eine Scharnierelement ist so mit den beiden Wangen verbunden, dass beide Wangen eine Scherbewegung relativ zueinander ausführen, sich also in ihrer Längsrichtung relativ zueinander verschieben können. Durch die zugfeste Ausgestaltung des Scharnierelements ist dabei sichergestellt, dass die beiden Verankerungspunkte des Scharnierelements in den Wangen stets den gleichen Abstand voneinander haben, also der eine Verankerungspunkt einen Kreis um den anderen Verankerungspunkt im Zuge der Scherbewegung beschreibt. Aufgrund der zugfesten Verbindung am distalen Ende führt die Scherbewegung zu einer Krümmung der Wangen. Ausdrücklich soll eine kontrollierte Elastizität des Scharnierelements in dessen Längsrichtung gestattet sein.

Die andere Wange ist wenigstens biegesteif, überträgt also wenigstens Druckkräfte. Lediglich in einer Weiterbildung kann sie auch zusätzlich zugfest sein.

Die Vorrichtung mit den beiden am proximalen Ende festgelegten Wangen nach den obigen Druckschriften kann nach der Lehre der EP-A-1203640 bei einer Zange eingesetzt werden.

In der DE 10 2007 026 721 A1 ist ein Werkzeug beschrieben, insbesondere ein medizinisches Greifwerkzeug mit wenigstens zwei Branchen, insbesondere zum Greifen, Halten, Bearbeiten oder Untersuchen von Körperbestandteilen, bei dem wenigstens eine der Branchen insbesondere bei einer Bewegung verformbar ist.

Die Einsatzmöglichkeiten der oben beschriebenen, bekannten Vorrichtungen sind begrenzt. Die Erfindung setzt sich daher zum Ziel, den bekannten Grundaufbau so abzuändern, dass die Einsatzmöglichkeiten vergrößert werden.

Diese Aufgabe wird erfindungsgemäß durch ein Halte- und/oder Spreizwerkzeug nach Anspruch 1 gelöst.

Durch diese einfache Lösung ist das Einsatzgebiet des biegsamen Manipulatorwerkzeugs gegenüber den vorbekannten Vorrichtungen deutlich erweitert, weil die lineare Antriebsbewegung direkt durch mit der Wange verbundene Zugmittel oder Druckstangen erfolgen kann. Dies führt zu einem geringeren Platzbedarf, so dass sich das Manipulatorwerkzeug insbesondere für kompakte und dünne, langgestreckte Werkzeuge eignet. Die Längsbewegung der antreibbaren Wange führt zu einer kontrollierten und steuerbaren Verformung des Manipulatorwerkzeuges.

Der längsbewegliche Antrieb wenigstens einer Wange am proximalen Ende ist zum Antrieb eines Unterwasserfahrzeugs, wie es in der WO-A-2008/128780 beschrieben ist, zwar bekannt; mit dem erfindungsgemäßen Gegenstand wird dieser Antrieb nunmehr zur Betäti
gung eines Werkzeuges eingesetzt, wobei als Werkzeug im Sinne der Erfindung auch ein motorisch oder manuelle betätigter Werkzeugträger anzusehen ist.

Die erfindungsgemäße Lösung kann durch zusätzliche, beliebig miteinander kombinierbare Merkmale weiter verbessert werden, wie nachstehend beschrieben ist.

So kann in einer vorteilhaften Ausgestaltung die längsbeweglich antreibbare Wange sowohl zugfest als auch biegesteif ausgebildet sein, so dass als Antriebskräfte sowohl Zug- als auch Druckkräfte auf die Wange einwirken können. In dieser Ausgestaltung ist die längsverschiebliche Wange vorzugsweise hin und her beweglich antreibbar am proximalen Ende gehalten.

Wird das Manipulatorwerkzeug als Werkzeugträger verwendet, so kann am distalen Ende oder an wenigstens einer Wange eine Werkzeughalter angeordnet sein. Bei dieser Ausgestaltung dient das Manipulatorwerkzeug dazu, dass in der Werkzeughalter angeordnete Werkzeug zu bewegen.

Um bei einer Verwendung als Werkzeughalter wenigstens ein Werkzeug im Inneren des Manipulatorwerkzeuges aufzunehmen, kann beispielsweise eine Wange hohl und/oder der Zwischenraum zwischen den Wangen als Aufnahme ausgestaltet sein. Zu diesem Zweck können die Scharnierelemente ringförmig, hohl und/oder gekrümmt sein.

Das Manipulatorwerkzeug kann an seinem proximalen Ende an einer Werkzeugbasis gelenkig gelagert oder fest eingespannt sein, wobei die Befestigung über wenigstens eine Wange oder ein Scharnierelement erfolgen kann. Dabei kann die als Befestigung dienende Wange an der Werkzeugbasis. Bei einer gelenkigen Lagerung oder einer hohen Flexibilität im an der Werkzeugbasis angrenzenden Bereich des Manipulatorwerkzeuges kann das gesamte Manipulatorwerkzeug durch den Antrieb der längsbeweglichen Wange verschwenkt werden, bis eine Wange an einen Gegenstand fährt. Erst dann setzt bei weiterem Antrieb der längsbeweglichen Wange eine Verformung aufgrund des vom Gegenstand ausgeübten Widerstandes ein.

Alternativ können auch beide, oder mehr als zwei Wangen am proximalen Ende längsbeweglich gehalten sein, so dass sich die durch die Antriebsbewegung hervorgerufene Verformung des Manipulatorwerkzeugs in Abhängigkeit von der Relativbewegung der beiden Wangen am proximalen Ende ergibt. Eine kontrollierte Verformung des Manipulatorwerkzeuges lässt sich erreichen, wenn wenigstens eine Wange eine in Richtung vom proximalen zum distalen Ende sich verändernde Steifigkeit aufweist. An Stellen erhöhter Steifigkeit wird sich das Manipulatorwerkzeug durch die Antriebsbewegung weniger stark krümmen als an Stellen verringerter Steifigkeit.

Die eine Wange, vorzugsweise die wenigstens biegesteife Wange, kann über die Verbindungsstelle mit der anderen Wange hinaus verlängert sein. Die Verlängerung kann insbesondere platten- oder scheibenförmig ausgestaltet sein, was insbesondere als Träger für Wisch-, Schab- und/oder Kratzorgane sinnvoll ist.

An das distale Ende kann sich auch Manipulatorwerkzeug und/oder eine Vorrichtung, wie sie aus der EP-A-1040999 bekannt ist, anschließen.

In einer weiteren vorteilhaften Ausgestaltung kann die wenigstens biegesteife Wange als Federelement ausgestaltet sein, dessen Federkraft in die wenigstens zugfeste Wange eingeleitet ist. Das Federelement dient bei dieser Ausgestaltung als Rückstellorgan, welches eine durch die Längsbewegung der antreibbaren Wange erzeugte Formänderung des Manipulatorwerkzeuges automatisch in eine Ausgangsstellung zurückführt. Die Federwirkung ist der Antriebswirkung entgegengesetzt.

Die Form der Wangen ist, solange der eingangs genannte Grundaufbau eingehalten ist, an den einzelnen Anwendungsfall anpassbar. So kann jede Wange klappen- oder scheibenförmig, gabelartig geteilt oder in Fingern auslaufend ausgestaltet sein. Ferner können die Wangen eine beliebige vorbestimme Krümmung aufweisen.

Das wenigstens eine Scharnierelement kann ebenfalls eine beliebige Raumform aufweisen. Das Halte- und/oder Spreizwerkzeug dient zum Halten, Greifen, Kneifen und/oder Klemmen etc. eines im Zwischenraum angeordneten Gegenstandes und in einer kinematischen Umkehr der Halte- oder Greiffunktion auch dazu, unter Vergrößerung des Zwischenraumes eine Spreizkraft auf zwei sich im Wesentlichen gegenüberliegende Wandabschnitte einer Öffnung oder eines Spaltes oder auf zwei voneinander beabstandete Gegenstände auszuüben. Bei der Halte- und Greiffunktion stellt die Arbeitsbewegung die Relativbewegung der beiden Backen aufeinander zu unter Verkleinerung des Zwischenraumes dar. Bei der Spreizfunktion bildet die Relativbewegung der beiden Backen voneinander weg die Arbeitsbewegung. In ihrem Grundaufbau müssen sich folglich ein erfindungsgemäßes Haltewerkzeug und ein erfindungsgemäßes Spreizwerkzeug zunächst nicht unterscheiden. Sollte jedoch die Haltefunktion in Einzelfällen gegenüber der Spreizfunktion die Abwandlung von Merkmalen erfordern, ist dies im Folgenden angegeben.

Ein beispielsweise in Längsrichtung des Manipulatorwerkzeuges ringförmig geschlossener Zwischenraum ergibt sich, wenn das Manipulatorwerkzeug an seinem distalen Ende mit dem distalen Ende der anderen Backe gelenkig verbunden ist.

Das Halte- und/oder Spreizwerkzeug kann insbesondere wenigstens ein Paar von sich bezüglich des Zwischenraumes gegenüberliegenden Manipulatorwerkzeugen in einer der obigen Ausgestaltungen als Backen aufweisen.

Das Manipulatorwerkzeug wie auch das Halte- und/oder Spreizwerkzeug kann aus einem integralen Körper geformt sein, beispielsweise durch ein Spritzguss-, Stanz- oder Schneideverfahren.

Zwei Manipulatorwerkzeuge können zur Bildung eines Halte- und/oder Spreizwerkzeuges an ihrem proximalen Ende einstückig miteinander verbundene antreibbare Wangen aufweisen. Bei dieser Ausführung erfolgt der Antrieb der beiden Manipulatorwerkzeuge gemeinsam und gleichzeitig. Die Antriebskraft kann in einer Weiterbildung dieser Ausführung durch ein zwischengeschaltetes Gelenk an die beiden gemeinsam antreibbaren Wangen geleitet sein, um Querkräfte zu vermeiden.

Eine handähnliche Ausgestaltung des Halte- und/oder Spreizwerkzeugs lässt sich erreichen, wenn an zumindest einer Seite des Zwischenraums eine Mehrzahl von Manipulatorwerkzeugen angeordnet ist. Das Manipulatorwerkzeug einer Seite übernimmt dabei die Funktion eines Fingers.

Die Manipulatorwerkzeuge können nicht nur, wie bei dem Halte- und/oder Spreizwerkzeug parallel zueinander, sondern auch in Reihe geschaltet sein. Hierzu kann wenigstens ein weiteres Manipulatorwerkzeug an wenigstens einer Wange eines anderen Manipulatorwerkzeuges befestigt sein. Das weitere Manipulatorwerkzeug kann am distalen Ende eines vorangehenden Manipulatorwerkzeuges angebracht sein. Alternativ oder zusätzlich können sich mehrere Manipulatorwerkzeuge auch eine gemeinsame, durchgehende Wange teilen. Die längsbeweglich antreibbare Wange kann sowohl zugfest als auch biegesteif ausgebildet sein, so dass eine Antriebsbewegung in beiden Richtungen unter Einleitung sowohl einer Zug- als auch einer Druckkraft möglich ist.

Am distalen Ende kann die eine Wange über die andere Wange hinaus unter Bildung eines Fortsatzes verlängert sein. Der Fortsatz kann als Werkzeughalter dienen oder selbst eine Werkzeugfunktion wahrnehmen. Vorzugsweise ist der Fortsatz von der wenigstens biegesteifen Wange gebildet. Die Form und Elastizität des Fortsatzes sowie sein Material können je nach Anwendungsfall variieren.

Das Manipulatorwerkzeug eignet sich insbesondere als endoskopisches Instrument in technischen oder medizinischen, insbesondere minimalinvasiven Anwendungen. Hierzu kann es innerhalb einer flexiblen Hülle angeordnet sein. Das erfindungsgemäße Halte- und/oder Spreizwerkzeug ist aufgrund des geringen Platzbedarfs ebenfalls insbesondere für endoskopische Anwendungen geeignet und kann insbesondere an der Spitze eines Endoskops eingesetzt werden. Die Antriebsmittel für die wenigstens eine längsverschiebliche Wange können durch das Endoskop geführt sein, was insbesondere bei der Verwendung von biegeschlaffen Zugmitteln als Antriebsmittel ohne großen Konstruktionsaufwand und ohne Beeinträchtigung der Beweglichkeit des Endoskops möglich ist.

Um die Flexibilität zu erhöhen, können gemäß einer vorteilhaften Ausgestaltung wenigstens drei Wangen vorgesehen sein, von denen wenigstens zwei längsbeweglich angetrieben sind. Die Wangen können unterschiedlich oder gleich lang und/oder miteinander verbunden sein.

Liegen gemäß einer Ausgestaltung wenigstens drei Wangen in einer gemeinsamen Manipulatorebene, so lässt sich eine ebene Verformung erzielen. Wirken die Antriebsbewegungen der wenigstens zwei längsbeweglich angetriebenen Wangen gegeneinander, so kann auf eine federnde Ausgestaltung der biegefesten Wange verzichtet werden.

Um eine kontrollierte räumliche Verformung zu erreichen, können die wenigstens drei Wangen in unterschiedlichen Manipulatorebenen liegen. Die Manipulatorebenen können sich insbesondere in einer gemeinsamen Achse schneiden, wobei diese Achse bevorzugt in Längsrichtung des Manipulatorwerkzeuges weist. So lassen sich beispielsweise bei drei um 120 Grad versetzten Wangen beliebige Krümmungen in jede Raumrichtung erzeugen.

Jeweils benachbarte Wangen können durch Scharnierelemente verbunden sein, so dass eine kombinierte Struktur mit komplexer Verformungsgeometrie entsteht.

Mehrere Wangen können aber auch um einen Innenraum herum angeordnet sein, so dass ein schlauchförmiges Gebilde entsteht., wobei die Scharnierelemente in Umfangsrichtung und die Wangen in Längsrichtung den Schlauch aufspannen können. Der Innenraum kann in distaler Richtung offen sein, so dass Fluide und/oder Werkzeuge am distalen Ende appliziert werden können. Wenn der Innenraum auch am proximalen Ende geöffnet ist, können durch den Innenraum Fluide hindurchgeleitet werden. Hierzu ist das Manipulatorwerkzeug vorzugsweise mit einer fluiddichten, schlauchförmigen Hülle umgeben.

Einzelne Wangen des Schlauches können am proximalen Ende unabhängig voneinander längsbeweglich angetrieben sein. Die längsbeweglichen Wangen können auch mit einem steifen Ring am proximalen Ende miteinander verbunden sein, so dass sich die Bewegung des Ringes, insbesondere sein Verkippen, in eine Antriebsbewegung aller damit verbundenen Wangen umsetzt und eine Verformung des Schlauches bewirkt. Auf diese Weise erhält man ein eigenständiges schlauchförmiges, aktiv steuerbares Instrument, das beispielsweise als Tubus in der Medizintechnik einsetzbar ist.

Jeweils eine wenigstens biegesteife Wange kann mit jeweils wenigstens zwei wenigstens zugfesten, längsbeweglichen Wangen am distalen Ende verbunden sein, die in einer Manipulatorebene an jeweils einer Seite der wenigstens biegesteifen Wange liegen und somit einander entgegenwirken.

Die wenigstens biegesteife Wange kann auch hohl sein, so dass sie selber einen Schlauch bildet oder einen Schaft für die Aufnahme von Instrumenten.

Schließlich muss die Verbindung der Wangen am distalen Ende nicht keilförmig verlaufen. Wesentlich ist lediglich, dass sich im Zuge der Verformung am distalen Ende der Verbindungswinkel zwischen den beiden Enden nicht ändert, die Verbindung also im Wesentlichen winkelstarr ist.

Im Folgenden wird die Erfindung anhand von Ausführungsformen beispielhaft näher erläutert. Bei den einzelnen Ausführungsformen unterschiedliche Merkmale können dabei Maßgabe der obigen Ausführungen beliebig miteinander kombiniert werden, falls es bei speziellen Anwendung auf den mit dem Merkmal verbundenen Vorteil besonders ankommt.

Es zeigen:
- Fig. 1: eine schematische Darstellung einer ersten Ausführungsform des Manipulatorwerkzeuges in einer Ausgangsstellung;
- Fig. 2: eine schematische Darstellung der Ausführungsform der Fig. 1 in einer verformten Stellung;
- Fig. 3: eine weitere Ausführungsform des Manipulatorwerkzeuges in einer Ausgangsstellung;
- Fig. 4: die Ausführungsform der Fig. 3 in einer schematischen Darstellung einer verformten Stellung;
- Fig. 5: eine schematische Darstellung einer weiteren Ausführungsform des Manipulatorwerkzeuges in einer Ausgangsstellung;
- Fig. 6: die Ausführungsform der Fig. 5 in einer schematischen Darstellung einer verformten Stellung;
- Fig. 7 bis 10: schematische Darstellung von verschiedenen Ausführungsformen eines distalen Endes des Manipulatorwerkzeuges;
- Fig. 11: eine schematische Darstellung einer Ausführungsform eines Scharnierelements des Manipulatorwerkzeuges;
- Fig. 12: eine schematische Darstellung einer weiteren Ausführungsform des Manipulatorwerkzeuges;
- Fig. 13: eine schematische Darstellung einer Ausführungsform eines erfindungsgemäßen Halte- und/oder Spreizwerkzeuges;
- Fig. 14: eine schematische Darstellung einer weiteren Ausführungsform eines erfindungsgemäßen Halte- und/oder Spreizwerkzeuges in einer Ausgangsstellung;
- Fig. 15: eine schematische Darstellung der Ausführungsform der Fig. 14 in einer verformten Stellung;
- Fig. 16: eine schematische Darstellung einer weiteren Ausführungsform eines erfindungsgemäßen Halte- und/oder Spreizwerkzeuges;
- Fig. 17: eine schematische Darstellung einer weiteren Ausführungsform eines erfindungsgemäßen Halte- und/oder Spreizwerkzeuges in einer Ausgangsstellung;
- Fig. 18: eine schematische Darstellung der Ausführungsform der Fig. 17 in einem verformten Zustand;
- Fig. 19: eine schematische Darstellung einer weiteren Ausführungsform eines schematischen Halte- und/oder Spreizwerkzeuges;
- Fig. 20: eine schematische Darstellung einer weiteren Ausführungsform eines erfindungsgemäßen Halte- und/oder Spreizwerkzeuges;
- Fig. 21: eine schematische Darstellung einer Abwandlung des Halte- und/oder Spreizwerkzeuges der Fig. 20;
- Fig. 22: eine schematische Darstellung der Fig. 20;
- Fig. 23: eine schematische Darstellung einer weiteren Ausführungsform eines erfindungsgemäßen Halte- und/oder Spreizwerkzeuges;
- Fig. 24: eine schematische Darstellung der Ausführungsform der Fig. 23 in einer ausgelenkten Stellung;
- Fig. 25: eine schematische Darstellung einer weiteren Ausführungsform eines erfindungsgemäßen Halte- und/oder Spreizwerkzeuges;
- Fig. 26: eine schematische Darstellung eines Manipulatorwerkzeuges für endoskopieähnliche Anwendungen;
- Fig. 27: eine schematische Darstellung einer weiteren Ausführungsform eines Manipulatorwerkzeuges, ebenfalls insbesondere für endoskopieähnliche Anwendungen;
- Fig. 28: eine schematische Darstellung einer weiteren Ausführungsform eines Manipulatorwerkzeuges, insbesondere für endoskopische Anwendungen;
- Fig. 29: eine schematische Darstellung der Ausführungsform der Fig. 28 in einer ausgelenkten Stellung;
- Fig. 30: eine schematische Darstellung einer Schnittansicht entlang der Linie XXX-XXX der Fig. 28;
- Fig. 31: eine schematische Darstellung einer weiteren Ausführungsform eines Manipulatorwerkzeuges in einem ersten Betriebszustand;
- Fig. 32: eine schematische Darstellung der Ausführungsform der Fig. 31 in einer weiteren Betriebsstellung;
- Fig. 33: bis 35 schematische Darstellungen weiterer Ausführungsformen Manipulatorwerkzeuge;
- Fig. 36: eine schematische Darstellung einer weiteren Ausführungsform des Manipulatorwerkzeuges;
- Fig. 37: eine schematische Schnittdarstellung durch die Ausführungsform der Fig. 36;
- Fig. 38: eine schematische Darstellung einer manuellen Handhabe zur Betätigung eines Manipulatorwerkzeuges und/oder eines erfindungsgemäßen Halte- und/oder Spreizwerkzeuges;
- Fig. 39: eine schematische Darstellung einer weiteren Ausführungsform des Manipulatorwerkzeuges;
- Fig. 40: eine schematische Darstellung einer weiteren Ausführungsform des Manipulatorwerkzeuges;
- Fig. 41 bis 43: schematische Darstellungen von Abwandlungen einer weiteren Ausführungsform des Manipulatorwerkzeuges;
- Fig. 44: eine schematische Darstellung einer weiteren Ausführungsform eines Manipulatorwerkzeuges.

Bei der nachstehenden Beschreibung der erfindungsgemäßen Ausführungsformen werden für hinsichtlich Funktion und/oder Form entsprechende Bestandteile dieselben Bezugszeichen verwendet. Der Kürze halber ist bei den einzelnen Ausführungsformen lediglich auf Unterscheidungsmerkmale zu den übrigen Ausführungsformen eingegangen, wobei die Unterscheidungsmerkmale verschiedener Ausführungsformen miteinander kombiniert werden können.

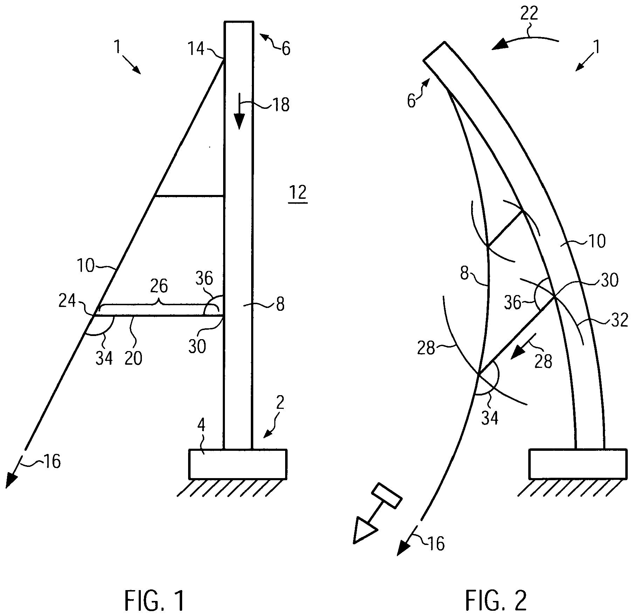

Zunächst wird der Aufbau eines Manipulatorwerkzeuges mit Bezug auf die Fig. 1 beschrieben.

Das Manipulatorwerkzeug 1 ist an seinen proximalen Ende 2 an einer lediglich schematisch dargestellten Werkzeugbasis 4, beispielsweise einer Werkzeughalter oder einem Gehäuse befestigt. Das distale Ende 6 ist frei beweglich. Das Manipulatorwerkzeug 1 weist zwei Wangen 8, 10 auf, von denen die eine Wange 8 wenigstens biegefest und biegesteif und die andere Wange 10 wenigstens zugfest ist. Die wenigstens zugfeste Wange 10 kann aus einem biegeschlaffen Zugmittel, wie beispielsweise einem Seil, einem Riemen, einem Band oder einer Kette gebildet sein.

Beide Wangen 8, 10 sind wenigstens in der Manipulationsebene 12, die in Fig. 1 der Zeichenebene entspricht, flexibel, so dass das gesamte Manipulatorwerkzeug 1 biegsam ist. Die wenigstens biegesteife Wange 8 kann die Funktion eines Federelementes übernehmen. Die beiden Wangen 8, 10 sind am proximalen Ende 2 voneinander beabstandet und am distalen Ende 6 miteinander verbunden. Die Verbindung 14 der beiden Wangen 8, 10 ist so ausgestaltet, dass eine in der wenigstens zugfesten Wange 10 herrschende Zugkraft 16 in die wenigstens biegesteife Wange 8 eingeleitet wird. Zwischen ihren jeweiligen Enden am proximalen Ende 2 und dem Verbindungspunkt 14 am distalen Ende 6 verlaufen die beiden Wangen 8, 10 nebeneinander in einem keilförmigen Winkel von weniger als 90 Grad, so dass die Zugkraft 16 über die Verbindung 14 zu einer Druckkraft 18 in der wenigstens biegesteifen Wange führt.

Zwischen dem proximalen Ende 2 und dem distalen Ende 6 ist wenigstens ein zumindest zugfestes Scharnierelement 20 angeordnet, das die beiden Wangen 8, 10 miteinander verbindet. Vorzugsweise ist eine Mehrzahl von Scharnierelementen vorhanden. Das Scharnierelement 20 kann gelenkig mit wenigstens einer Wange 8, 10 verbunden sein und/oder ausreichende Flexibilität besitzen, um der Scherbewegung zu folgen. Das Scharnierelement 20 kann biegeschlaff sein.

Zwar kann grundsätzlich jede der beiden Wangen 8, 10 unabhängig voneinander sowohl zugfest als auch biegesteif ausgestaltet sein, dies ist jedoch im Allgemeinen mit einem größeren Gewicht des Manipulatorwerkzeuges verbunden, als bei einer Auslegung, welche die im Betrieb in den Wangen 8, 10 auftretenden Kräfte berücksichtigt.

Mit Bezug auf die Fig. 1 und 2 wird nun die Funktion des Manipulatorwerkzeuges 1 beschrieben.

Bei der Ausführungsform der Fig. 1 ist die eine Wange 10 längsverschieblich am proximalen Ende 2 gelagert. Wird eine Zugkraft 16 in die Wange 10 geleitet, so bewegt sich die Wange zum proximalen Ende 2 in ihrer Längsrichtung weg vom distalen Ende 6 und die Zugkraft 16 biegt das distale Ende 6 in ihre Richtung, wie in Fig. 2 durch den Pfeil 22 angedeutet ist.

Im Zuge der Verformung bewegt sich der Verankerungspunkt 24, an dem das Scharnierelement 20 an der wenigstens zugfesten Wange 10 befestigt ist, auf einer von der Länge 26 des Scharnierelements 20 bestimmten Kreisbahn 28 um den Verankerungspunkt 30 des Scharnierelements 20 an der wenigstens biegesteifen Wange. Umgekehrt bewegt sich natürlich auch der Verankerungspunkt 30 entlang einer Kreisbahn 32 um den Verankerungspunkt 24. Durch Veränderung der Länge 26 und des Winkels 34, 36 zwischen dem Scharnierelement 20 und den Wangen 8, 10 in der Grundkonstruktion lässt sich die Verformungsgeometrie des Manipulatorwerkzeuges beeinflussen.

Wie der Vergleich der Fig. 1 und 2 zeigt, sind die Wangen 8, 10 über das wenigstens eine Scharnierelement 20 so verbunden, dass sie gegeneinander scherbeweglich sind, also eine Relativbewegung zwischen den beiden Wangen in ihrer Längsrichtung vom proximalen zum distalen Ende 2, 6 möglich ist. Diese Scherbewegung zeigt sich in der Veränderung der Winkel 34, 36 im Zuge der Verformung.

Die Längsbewegung der Wange 10, die die Verformung des Manipulatorwerkzeuges bewirkt, kann auf vielfältige Weise erfolgen. Beispielsweise kann sich die Wange 8 in einem Zugmittel fortsetzen, das über eine angetriebene Wickelrolle geführt ist.

Lässt die Zugkraft nach, so nimmt das Manipulatorwerkzeug aufgrund der Federwirkung der wenigstens biegesteifen Wange 8 wieder die Ruheposition ein.

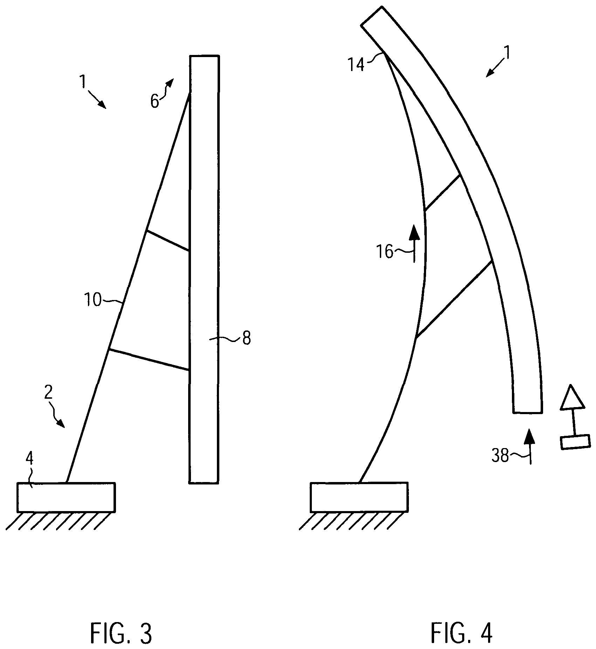

Eine Modifikation der Ausführungsform der Fig. 1 und 2 ist in den Fig. 3 und 4 gezeigt.

Im Unterschied zu der Ausführungsform der Fig. 1 und 2 ist bei der Ausführungsform der Fig. 3 und 4 die wenigstens biegesteife Wange 8 längsbeweglich ausgestaltet, während die wenigstens zugfeste Wange 10 am distalen Ende 6 beispielsweise über die Werkzeugbasis 4 festgelegt ist. Zur Verformung des Manipulatorwerkzeuges wird eine Druckkraft 38 in die Wange 10 eingeleitet, die über die Verbindung 14 in einer Zugkraft 16 in der wenigstens zugfesten Wange 10 resultiert.

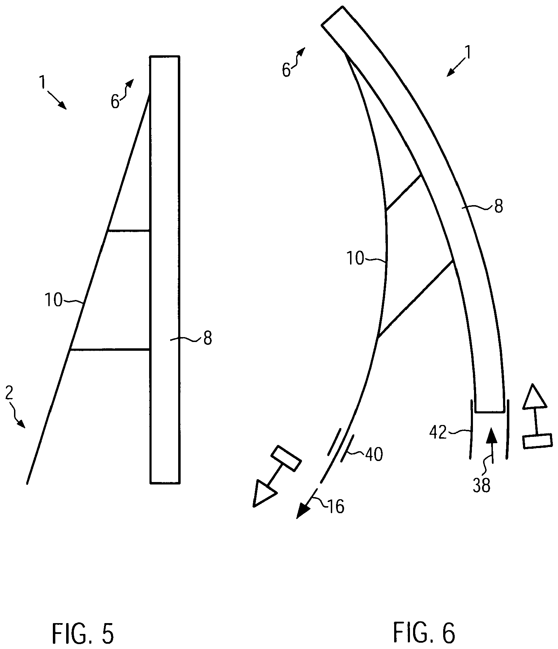

Eine weitere Ausführungsform des Manipulatorwerkzeuges 1 ist in den Fig. 5 und 6 dargestellt. Die Ausführungsform der Fig. 5 und 6 ist am proximalen Ende 2 schwimmend gelagert, so dass gleichzeitig in die wenigstens biegesteife Wange 8 eine Druckkraft 38 und in die wenigstens zugfeste Wange 10 eine Zugkraft eingeleitet werden kann.

Wird eine der beiden Wangen 8, 10 festgelegt und nur die andere Wange 10, 8 bewegt, so ergeben sich wieder die Ausführungen der Fig. 1 und 2 (bei Festlegung der Wange 8) bzw. 3 und 4 (bei Festlegung der Wange 10).

Um die Einleitung von Momenten in die Wangen 8, 10 zu vermeiden und in die Wange 8 eine reine Druckkraft 38 bzw. in die Wange 10 eine reine Zugkraft 16 einzuleiten, können Linearführungen 40, 42 vorgesehen sein, welche die jeweilige Wange 8, 10 geradlinig in deren Längsrichtung führen.

Sind beide Wangen 8, 10 sowohl zugfest als auch drucksteif bzw. beide Wangen biegesteif, so kann eine Wange am proximalen Ende 2 an der Werkzeugbasis 4 festgelegt werden und in die andere Wange sowohl eine Zugkraft 16 als auch eine Druckkraft 38 eingeleitet werden, so dass eine Verformung des biegsamen Manipulatorwerkzeuges sowohl mit der in Fig. 4 dargestellten Krümmung als auch mit der hierzu entgegengesetzten Krümmung erzielen.

Natürlich kann auch das gesamte Manipulatorwerkzeug 1 an der Werkzeugbasis 4 längsverschieblich oder schwenkbar gehalten sein.

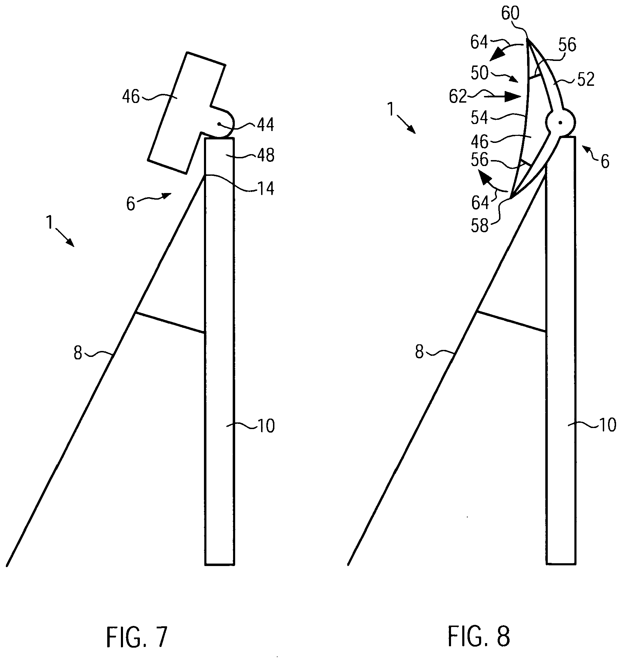

In den vorangegangenen Ausführungsformen lässt sich das Manipulatorwerkzeug 1 insbesondere als Werkzeugträger einsetzen. Dies ist kurz anhand der Ausführungsformen der Fig. 7 bis 10 beschrieben.

Bei der Ausführungsform der Fig. 7 ist am distalen Ende 6 ein Werkzeughalter 44 angebracht, der ein Werkzeug 46 tragen kann.

Wie Fig. 7 zeigt, kann der Werkzeughalter 44 an einem über den Verbindungspunkt 14 der beiden Wangen 8, 10 sich fortsetzenden Abschnitt 48, im Folgenden als Fortsatz bezeichnet, angebracht sein. Bei der Bewegung 22 (Fig. 2) des Manipulatorwerkzeuges kann das Werkzeug 46 in Anschlag gegen ein hier nicht dargestelltes Werkstück gebracht werden.

Fig. 8 zeigt eine Variante der Fig. 7, bei der als Werkzeug 46 eine Abwandlung 50 des Manipulatorwerkzeuges 1 in Einsatz kommt, die im Unterschied zum Manipulatorwerkzeug 50 passiv ausgelenkt wird.

Die Abwandlung 50 weist ebenfalls eine wenigstens biegesteife Wange 52 und eine wenigstens zugfeste Wange 54 auf, die über wenigstens ein Scharnierelement 56 miteinander verbunden sind.

Die Abwandlung 50 ist im Mittenbereich der wenigstens biegesteifen Wange 52 gelenkig am Werkzeughalter 44 befestigt, so dass sich die wenigstens zugfeste Wange 54 zwischen den beiden bogenförmig aufgespannten Enden der wenigstens biegesteifen Wange 52 erstreckt.

Übt ein in Fig. 8 nicht dargestellter Gegenstand eine Druckkraft 62 auf die wenigstens zugfeste Wange 54 des Werkzeuges 46 aus, so gibt die wenigstens zugfeste Wange 54 an dieser Stelle nach. Die unbelasteten Enden bewegen sich gleichzeitig in einer Bewegung 64 in Richtung des Angriffspunktes der Druckkraft 62 und versuchen, den kraftausübenden Gegenstand zu umgreifen. Auf diese Weise schmiegt sich die Abwandlung 50 selbsttätig an einen Gegenstand an.

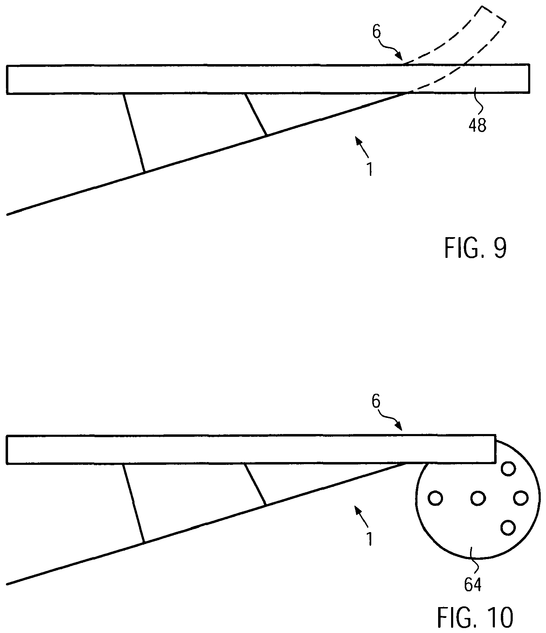

Bei der Ausführungsform der Fig. 9 ist der Fortsatz 48 elastisch nachgiebig gestaltet und kann beispielsweise zur Aufnahme von Reinigungs- und/oder Kratzwerkzeugen verwendet werden. In Fig. 10 ist am Manipulatorwerkzeug 1 eine flächige, beispielsweise scheiben- oder plattenförmige Erweiterung 64 vorgesehen, welche ebenfalls Träger von Kratz- und/oder Reinigungswerkzeugen sein kann.

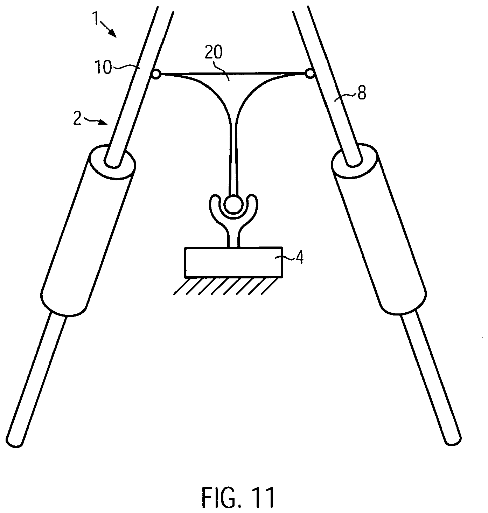

Fig. 11 zeigt eine weitere Ausführungsform des Werkzeugträgers 1, bei dem nur das proximale Ende 2 dargestellt ist. Bei der Ausführungsform der Fig. 11 ist das Manipulatorwerkzeug 1 über ein Scharnierelement 20 an der Werkzeugbasis 4 befestigt. Diese Ausgestaltung eignet sich insbesondere für die Ausführungsform der Fig. 5 und 6, wobei beide Wangen 8, 10 längsbeweglich antreibbar sind. Die Befestigung des Scharnierelements 20 kann je nach gewünschter Verformungsgeometrie und Belastungssituation im Anwendungsfall, wie in Fig. 11 dargestellt, momentenfrei erfolgen; das Scharnierelement 20 kann aber auch an der Werkzeugbasis 4 fest eingespannt sein.

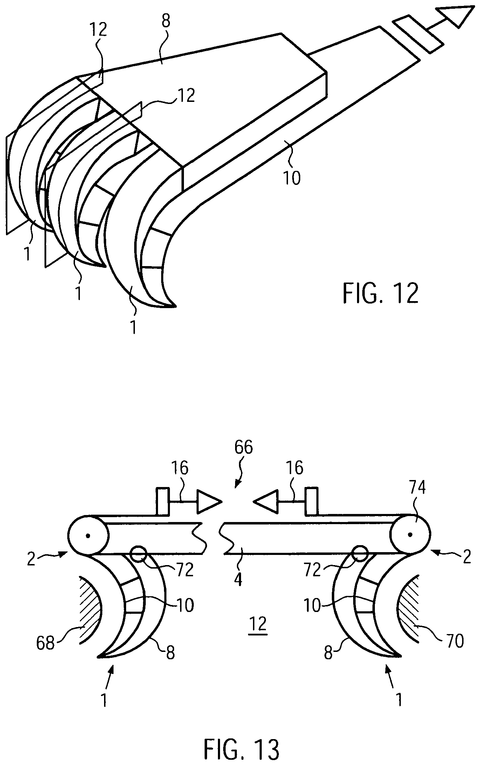

Wie die Ausführungsform der Fig. 12 zeigt, können auch mehrere Manipulatorwerkzeuge 1 nebeneinander angeordnet und unabhängig oder gleichzeitig miteinander betätigt werden. Die Manipulatorebenen 12 können parallel verlaufen oder sich schneiden. Beispielsweise können sich die beiden Wangen 8, 10 fingerförmig auffächern, oder die einzelnen Finger von jeweils einem Manipulatorwerkzeug 1 gebildet sein. Im Bereich der Handwurzel kann, wenn dies gewünscht ist, die biegesteife Wange eine hohe Biegesteifigkeit aufweisen oder starr ausgebildet sein, um eine Verformung in diesem Bereich zu verringern oder zu verhindern und die Verformung im Wesentlichen auf den Bereich der Finger zu begrenzen.

Das Manipulatorwerkzeug 1 lässt sich somit auch als Greifwerkzeug einsetzen.

Fig. 13 zeigt eine weitere Ausgestaltung, in dem zwei Manipulatorwerkzeuge 1 parallel zueinander angeordnet sind, wobei die proximalen Enden 2 an einer gemeinsamen Werkzeugbasis 4 angeordnet und die wenigstens biegesteifen Wangen einander zugeordnet sind. Manipulatorwerkzeuge 1 liegen in wenigstens nahezu derselben Manipulatorebene 12, in Fig. 13 der Zeichenebene.

Aus der Anordnung gemäß Fig. 13 resultiert ein Spreiz- und/oder Greifwerkzeug 66, das zwischen zwei Gegenständen 68, 70 oder in einer Öffnung eines Gegenstandes verspreizt werden kann. Hierzu werden die im Ruhezustand vorzugsweise geraden oder zueinander gekrümmten Manipulatorwerkzeuge 1 zwischen die Gegenstände 68, 70 eingeführt und anschließend die Wangen 10 längsverschieblich angetrieben, indem an jeder Wange 10 eine Zugkraft 16 einwirkt. Darauf krümmen sich die Wangen 8 in Richtung der Gegenstände 68, 70 und das Halte- und/oder Spreizwerkzeug verkeilt sich zwischen den Gegenständen 68, 70.

Die beiden Manipulatorwerkzeuge 1 können entlang der Werkzeugbasis 4 aufeinander zu oder voneinander weg verschieblich angeordnet sein, so dass die Distanz zwischen den beiden Manipulatorwerkzeugen 1 verändert werden kann. Zusätzlich kann die Wange 8 über ein Gelenk 72 an der Werkzeugbasis 4 befestigt sein, so dass beim Aufbringen der Zugkraft 16 zunächst die Wange 8 im Wesentlichen unverformt gegen den jeweils ihr zugeordneten Gegenstand 68, 70 fährt und sich erst beim weiteren Aufbringen der Zugkraft während des Anliegens um den Gegenstand 68, 70 krümmt. Die Wange 10 oder ein mit ihr verbundenes Zugmittel kann über eine Umlenkrolle 74 umgelenkt sein.

Selbstverständlich können das Gelenk 72 und die Umlenkrolle 74 auch bei einer beliebigen anderen hier beschriebenen Ausführungsform verwendet sein.

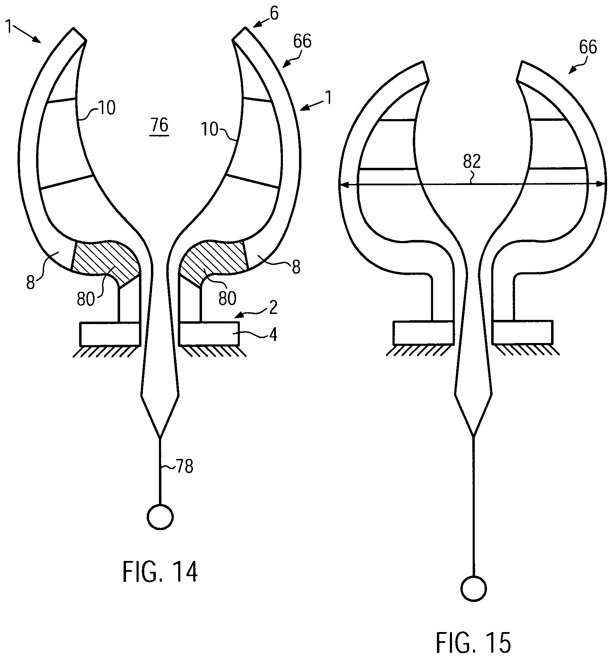

Fig. 14 und 15 zeigen eine weitere Ausführungsform eines Halte- und/oder Spreizwerkzeuges, das aus zwei Manipulatorwerkzeugen 1 aufgebaut ist, die sich bezüglich eines Zwischenraums 76, der beispielsweise der Aufnahme eines Werkstückes dient, gegenüberliegen. Bei der Ausführungsform der Fig. 14 sind die wenigstens zugfesten Wangen 10 einander zugewandt und begrenzen den Zwischenraum 76. Zum distalen Ende 6 hin ist der Zwischenraum 76 offen, so dass von dieser Seite ein Werkstück (nicht dargestellt) eingeführt werden kann.

Die beiden Wangen 8 sind am proximalen Ende 2 an der Werkzeugbasis 4 befestigt. Die Wangen 10 sind am proximalen Ende 2 zusammengeführt und enden in einem gemeinsamen Zugmittel 78, so dass sie bei Zug gleichzeitig betätigt werden.

Der Verlauf der Steifigkeit der Wangen 8 in Richtung vom proximalen Ende 2 zum distalen Ende 6 ist so gewählt, dass im Bereich des proximalen Endes des Zwischenraums 76 ein Bereich 80 verringerter Steifigkeit entsteht. Dies führt dazu, dass bei einem Zug 16 (Fig. 15) an den Wangen 10 sich der Bereich 80 stärker verformt als der übrige Bereich der Wange 8. Der Bereich 80 dient somit als elastisches Gelenk, um welche distalen Enden 6 der beiden Manipulatorwerkzeuge 1 schwenken.

Der Bereich 80 verringerter Steifigkeit, wie in Fig. 14 gezeigt, kann durch eine Formgebung erfolgen, bei der Spannungsspitzen im Material entstehen. Alternativ oder zusätzlich kann dieser Funktion durch eine verringerte Wandstärke oder durch Verwendung eines Materials geringerer Steifigkeit erzielt werden. Der Bereich 80 kann anstelle eines Gelenks 72 verwendet werden, wenn es auf eine elastische Rückstellfunktion ankommt.

Die beschriebene Halte- und/oder Greiffunktion der Ausführungsform der Fig. 14 lässt sich, wie im Übrigen bei den anderen Ausführungsformen auch, auf einfache Weise in eine Spreizfunktion umkehren, wenn die wenigstens biegesteifen Wangen 8 im unverformten Zustand die in Fig. 14 dargestellte Position einnehmen. Dann werden nämlich die Wangen 8 durch die Zugkraft 16 elastisch in Richtung aufeinander zu ausgelenkt und es verringert sich die Breite 82 des Halte- und/oder Spreizwerkzeuges, so dass es in eine Öffnung eingeführt werden kann. Wird die Zugkraft verringert, so versuchen sich die Wangen 8 wieder elastisch aufzuspreizen.

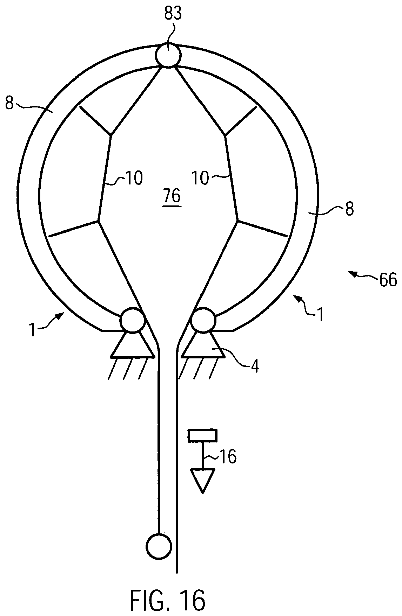

Die Ausführungsform der Fig. 16 unterscheidet sich von der Ausführungsform der Fig. 14 und 15 durch eine gelenkige Verbindung der distalen Enden 6 der beiden Manipulatorwerkzeuge 1, so dass ein ringförmig geschlossener Innenraum 76 entsteht. Diese Ausführungsform eignet sich in ihrer Grundform zum Aufspreizen, aber auch zum sicheren Halten von rohrförmigen Gegenständen.

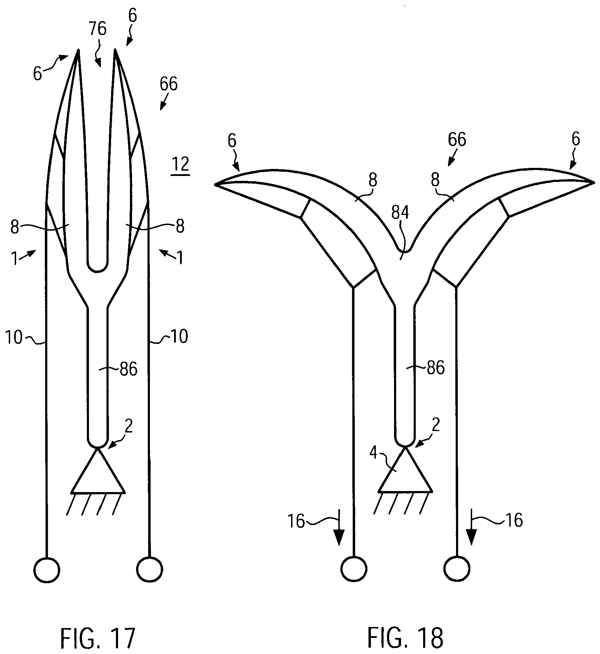

In den Fig. 17 und 18 ist ein weiteres Beispiel für eine Halte- und/oder Spreizvorrichtung 66 mit zwei Manipulatorwerkzeugen 1 gezeigt. Auch dieses Werkzeug kann je nach Vorkrümmung der biegesteifen Wangen 8 wahlweise als Spreiz- und/oder Haltewerkzeug eingesetzt werden.

Die beiden Wangen 8 sind bei der Ausführungsform der Fig. 17 und 18 an einer Wurzel 84, die wahlweise auch als Gelenk 72 ausgestaltet sein kann, miteinander verbunden. Die Wurzel 84 setzt sich in einem Stamm 86 fort, der mit der Werkzeugbasis 4 am proximalen Ende 2 verbunden ist. Die beiden wenigstens zugfesten Wangen 10 weisen voneinander weg und sind jeweils unabhängig voneinander längsverschieblich. Bei der in Fig. 17 gezeigten Ruhestellung verlaufen die beiden Wangen 8 nahezu parallel zueinander in der Manipulatorebene 12 und bilden zwischen sich den Zwischenraum 76. Bei Zug an den Wangen 10 bewegen sich die Wangen 8 unter Vergrößerung des Zwischenraumes 76 voneinander weg. In dieser Ausgestaltung ist das Halte- und/oder Spreizwerkzeug insbesondere zum beispielsweise schirmartigen Aufspannen bzw. Aufspreizen von Werkstücken zwischen den bezüglich des Zwischenraums 76 gegenüberliegenden distalen Enden 6 der beiden Manipulatorwerkzeuge 1 geeignet.

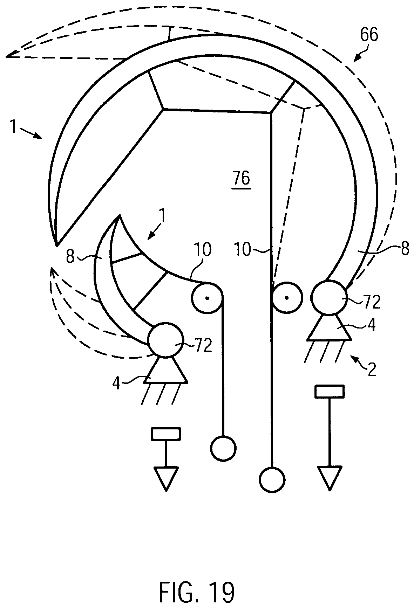

Schließlich lassen sich mit dem Halte- und/oder Spreizwerkzeug auch klauenähnliche Strukturen erzeugen, wie Fig. 19 zeigt. Zwei Manipulatorwerkzeuge 1 begrenzen mit ihren wenigstens zugfesten Wangen 10 einen Zwischenraum 76. Die biegesteifen Wangen 10 sind mittels Gelenken 72 mit der Werkzeugbasis 4 am proximalen Ende 2 des Halte- und/oder Spreizwerkzeuges 66 verbunden. Das eine Manipulatorwerkzeug 1 ist deutlich kleiner als das andere Manipulatorwerkzeug 1 und verschließt in mit durchgezogenen Linien dargestellten verformten Zustand den Zwischenraum 76, während sich das größere Manipulatorwerkzeug 1 mit seiner wenigstens zugfesten Wange 10 außen an die biegesteife Wange des kleineren Werkzeuges legt.

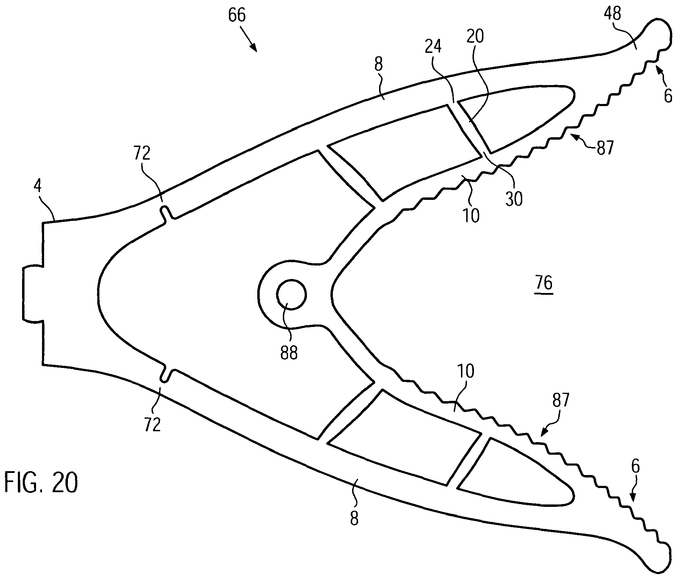

Mit Bezug auf die Fig. 20 ist eine aus einem Stück geformte Ausführungsform eines Halte- und/oder Spreizwerkzeuges 66 gezeigt, das mittels eines Spritzguss-, Stanz- oder Schneidverfahrens aus Plattenmaterial hergestellt sein kann.

Beide Wangen 8, 10 der die beiden Backen 87 bildenden Manipulatorwerkzeuge 1 sind biegesteif, wobei die dem Zwischenraum 76 zugewandten Wangen 10 eine geringere Wandstärke aufweisen als die äußeren Wangen 8. Die geringe Wandstärke 8 führt auch zu einer größeren Flexibilität der Wangen 10, so dass sie sich leichter um zu greifende Gegenstände legen.

Wie im Übrigen bei den anderen Ausführungsformen auch kann die den handzuhabenden Gegenständen zugewandte Oberfläche der die Halte- und/oder Spreizfunktion ausübenden Wangen je nach Bedarf strukturiert sein. So weisen bei der Ausführungsform der Fig. 20 die Wangen 10 eine Riffelung quer zur Längsrichtung auf.

Die scherbewegliche Verbindung über das wenigstens eine Scharnierelement 20 wird bei der Ausführungsform der Fig. 20 durch die Formgebung im Bereich der Verankerungspunkte 24, 30 erreicht. Aufgrund des nahezu rechtwinkligen Anschlusses der die Elemente 20 an die Wangen 8, 10 und der im Bereich der Verankerungspunkte 24, 30 leicht verringerten Wandstärke ist im Bereich der Verankerungspunkte 24, 30 die Beweglichkeit erhöht, so dass an diesen Stellen eine Gelenkfunktion erreicht wird.

Mit der Werkzeugbasis 4 sind die äußeren Wangen 8 über ein Gelenk 72 verbunden, das durch einen Schwächungsbereich in Form eines Einschnittes erzeugt ist.

Am distalen Ende 6 sind die Wangen 8, 10 miteinander wieder unter Bildung eines Fortsatzes 48 miteinander verbunden. Der Fortsatz 48 erlaubt ein sanftes Ergreifen von Gegenständen. An ihrem proximalen Ende sind die Wangen 10 einstückig miteinander verbunden. An der Stelle der Verbindung der beiden Wangen 10 kann ein Kupplungselement 88, beispielsweise in Form einer Öse, vorgesehen sein.

Das in Fig. 20 gezeigte Werkzeug 66 kann am Ende eines Manipulatorwerkzeuges 1, wie es in den Fig. 1 bis 6 gezeigt ist, eingesetzt werden. Werden in der Ausführungsform der Fig. 20 die Werkzeugbasis und die Kupplung 88 vertauscht oder werden an der Kupplung 88 Druckkräfte eingeleitet, so entsteht ein Spreizwerkzeug. Hierzu wäre es sinnvoll, die Riffelung nicht an den Wangen 10, sondern an den Wangen 8 vorzusehen.

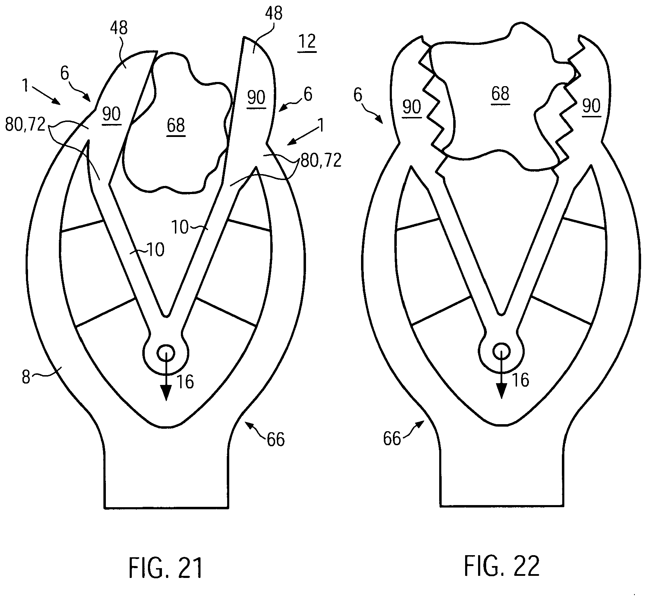

In Fig. 21 ist gezeigt, wie sich durch die Kombination zweier erfindungsgemäßer Manipulatorwerkzeuge 1 zu einem Halte- und/oder Spreizwerkzeug 66 ein Gegenstand 68 formadaptiv ergreifen lässt. Die Wangen 10 greifen infolge der Zugkraft 16 den Gegenstand 68 und passen sich an seine Außenkontur in der Manipulatorebene 12 an. Die Anpassung an die Kontur des Gegenstandes 68 überträgt sich auch auf einen Fortsatz 48, der zusammen mit einem am distalen Ende verstärkten Greifbereich 90 an der Innenseite der Wangen 10 einstückig ausgebildet ist. Die Formadaption kann durch die gelenkige Anbindung des Greifbereiches 90 über Gelenke 72 oder Bereiche 80 verringerte Biegesteifigkeit mit der äußeren Wange 8 und dem Rest der Wange 10 verbunden.

Fig. 22 zeigt, wie der Greifbereich 90 sich an die Kontur eines anderen Gegenstandes 68 aufgrund der gelenkigen Verbindung anpassen kann.

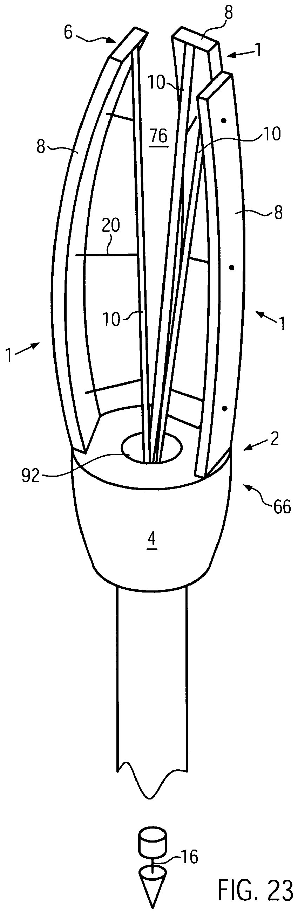

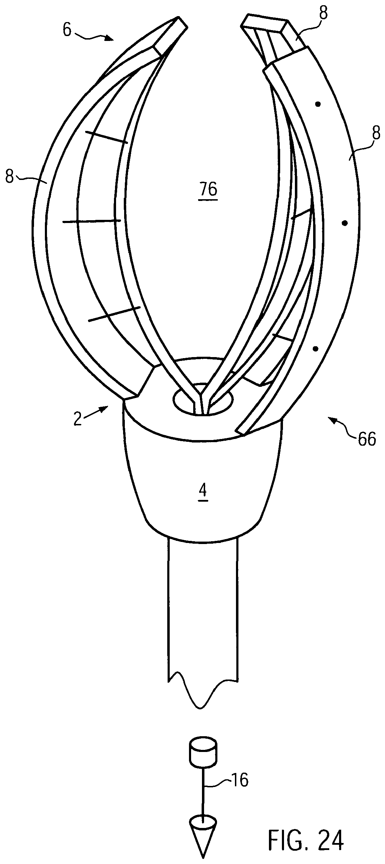

Eine Ausführungsform des Halte- und/oder Spreizwerkzeuges 66, die sich besonders für endoskopische Anwendungen eignet, ist in den Fig. 23 und 24 schematisch dargestellt. In der Fig. 23 ist das Halte- und/oder Spreizwerkzeug geschlossen, in der Fig. 24 in einer Greifstellung ohne gegriffenen Gegenstand dargestellt.

Das Halte- und/oder Spreizwerkzeug 66 weist drei oder mehr Manipulatorwerkzeuge 1 auf, die einen Innenraum 76 sternförmig umgeben und die innerhalb einer im Wesentlichen kreisförmigen Außenkontur angebracht sind. In dieser Ausgestaltung lässt sich das Halte- und/oder Spreizwerkzeug 66 beispielsweise in Verbindung mit Endoskopen verwenden und durch diese hindurchschieben.

An der Außenseite des Halte- und/oder Spreizwerkzeuges 66 sind die wenigstens biegesteifen Wangen 8 fest an der Werkzeugbasis 4 eingespannt angeordnet. Die Einspannung erfolgt hier über elastisch vorgespannte Gelenke, die eine die Wangen 8 voneinander weg bewegende Federkraft erzeugen. Die wenigstens zugfesten Wangen 10 sind dem Zwischenraum 76 zugewandt. Durch eine zentrale Öffnung 92 in der vorzugsweise nicht über die Außenkontur der Manipulatorwerkzeuges 1 ragenden Werkzeugbasis 4 kann eine Zugkraft 16 auf die wenigstens zugfesten Wangen 10 aufgebracht werden.

Ist kein Gegenstand im Zwischenraum 76 angeordnet, so bewirkt eine Zugkraft 16, dass die Manipulatorwerkzeuge 1 im wesentlichen unverformt gegen die Federkraft der elastisch vorgespannten Gelenke aufeinander zu bewegt werden und die in Fig. 23 dargestellte Stellung einnehmen und auch knospenförmig vollständig geschlossen sein können. Dies setzt allerdings voraus, dass die Steifigkeit im Gelenk geringer ist, als die Steifigkeit der biegesteifen Wangen. In der geschlossenen Stellung weist die Ausführungsform der Fig. 23 und 24 eine schlanke Querschnittsform auf, so dass das Greifwerkzeug besonders in beengten Raumsituationen eingesetzt, beispielsweise als Obstpflücker zwischen Ästen oder am Ende eines Endoskops zwischen Organen hindurchgeführt werden kann.

Befindet sich im geöffneten Zustand ein Gegenstand im Zwischenraum 76, so werden bei Aufbringen einer Zugkraft zunächst die Manipulatorwerkzeuge 1 unverformt einwärts bis zur Anlage an den Gegenstand verschwenkt. Erst die weitere Längsbewegung der wenigstens zugfesten Wangen 10 bewirkt die in Fig. 2 gezeigte Verformung der Manipulatorwerkzeugebzw. Biegesteifen Wangen 8, die sich dabei an die Außenkontur des Gegenstandes anlegen.

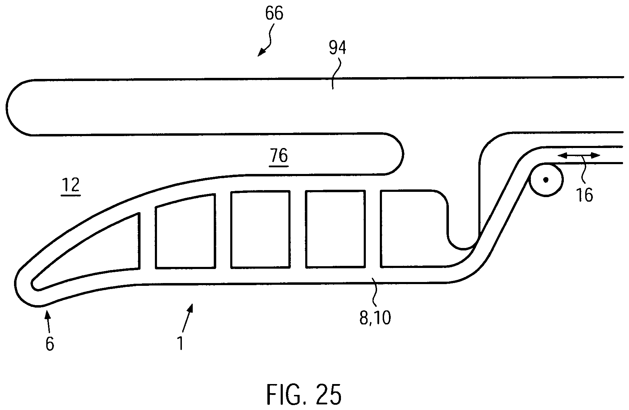

So kann nach der Ausführungsform der Fig. 25 das Manipulatorwerkzeug 1 eine Ausgangsstellung haben, in der es gegen die Backe 94 drückt. Durch Zug an der äußeren Wange 10 nimmt es die in Fig. 25 dargestellte Stellung ein, in der es von der gegenüberliegenden Backe 94 wegbewegt ist, so dass ein Gegenstand in den Zwischenraum 76 eingeführt werden kann.

Die Ausgestaltung der Backe 94 kann je nach gewünschter Funktion erfolgen. Bei einer geeigneten Greiffunktion kann die Backe 94 eine Anlagefläche für einen im Zwischenraum 76 zu haltenden Gegenstand ausbilden. Die Backe 94 kann jedoch auch als Klinge ausgestaltet sein, um den in dem Zwischenraum 76 angeordneten Gegenstand bei einem Abfall der Zugkraft 16 durchzuschneiden. Dies ist insbesondere im Bereich der Gefäßchirurgie von Nutzen, wenn das in Fig. 25 dargestellte Halte- und/oder Spreizwerkzeug beispielsweise in Endoskopen eingesetzt wird.

Die Ausführungsform der Fig. 25 kann bei entsprechend ergonomischer Ausgestaltung der Backe 94 und der Manipulatorwerkzeuges 1 auch als Handgriff eingesetzt werden, der über die Kraft 16 weitere nicht dargestellte Werkzeuge zu bedienen erlaubt.

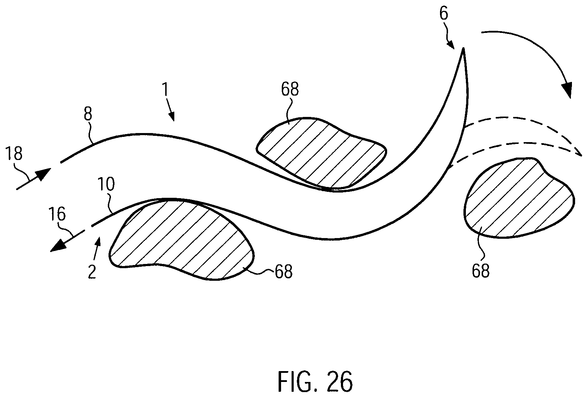

Fig. 26 zeigt ein Manipulatorwerkzeug 1, das als Endoskop eingesetzt wird und zwischen Gegenständen 68 hindurchgefädelt ist. Der Grundaufbau eines derartigen Endoskops kann dem Aufbau der Ausführungsform der Fig. 5 und 6 entsprechen, in dem die beiden Wangen 8, 10 biegesteif sind und sowohl Zug- als auch Druckkräfte aufnehmen, oder aber die eine Wange 8 nur Druckkräfte und die andere Wange 10 nur Zugkräfte aufnimmt. Solange das distale Ende 6 des Manipulatorwerkzeuges 1 frei ist, kann durch Aufbringen der Kräfte 16, 18 die Spitze bewegt und um die Gegenstände 68 herum gelegt werden. Auf diese Weise kann das distale Ende 6 stets so ausgerichtet werden, dass es zwischen zwei benachbarte Gegenstände 68 weist, und das Manipulatorwerkzeug 1 zwischengeschoben werden kann. In der Endposition kann ein am distalen Ende 6 angebrachtes Werkzeug oder ein Beobachtungsgerät um einen letzten Gegenstand 68 herum nochmals in Position gebracht oder dieser letzte Gegenstand ergriffen werden, wie durch die gestrichelte Linie in Fig. 26 angedeutet ist.

Die Scharnierelemente 20 sind in Fig. 26 der Übersichtlichkeit halber weggelassen.

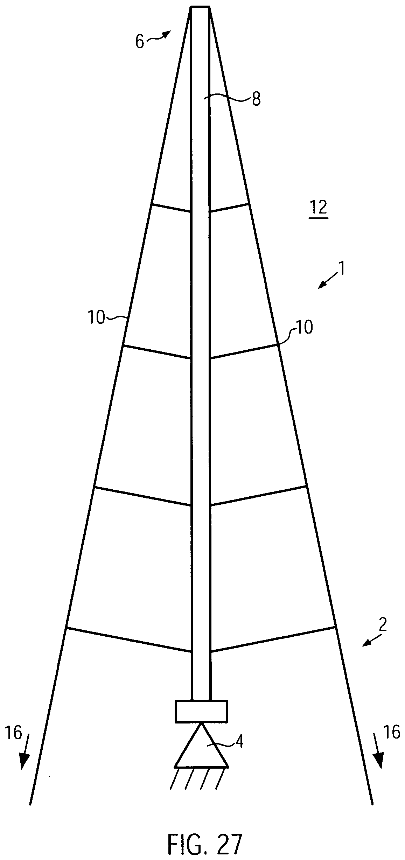

Die in Fig. 26 beschriebene Funktion lässt sich auch durch die in Fig. 27 gezeigte Ausgestaltung des Manipulatorwerkzeuges 1 erreichen, bei dem eine zentrale, biegesteife Wange 8 von wenigstens zwei wenigstens zugfesten Wangen 10 geteilt wird. Die zentrale biegesteife Wange 8 ist an der Werkzeugbasis 4 vorzugsweise schwenkbar gehalten. Jede einzelne Wange 10 ist an ihrem proximalen Ende 2 längsverschieblich antreibbar. Durch den Zug an einer der Wangen 10 kann eine Krümmung der biegesteifen Wange 8 in die jeweils gewünschte Richtung erreicht werden. Dabei können die Wangen 10 auch in mehr als einer Manipulatorebene 12 angeordnet sein und somit eine dreidimensionale Krümmung der zentralen Wange 8 bewirken. Auch dies ist für eine endoskopische Anwendung von Vorteil.

Da bei der Ausführungsform der Fig. 27 jeweils entgegenwirkende Paare von angetriebenen Wangen 10 vorgesehen sind, muss die wenigstens biegesteife Wange 8 nicht als Federelement ausgestaltet sein, da die Rückstellung jeweils durch Zug an der gegenüberliegenden Wange 10 erfolgen kann.

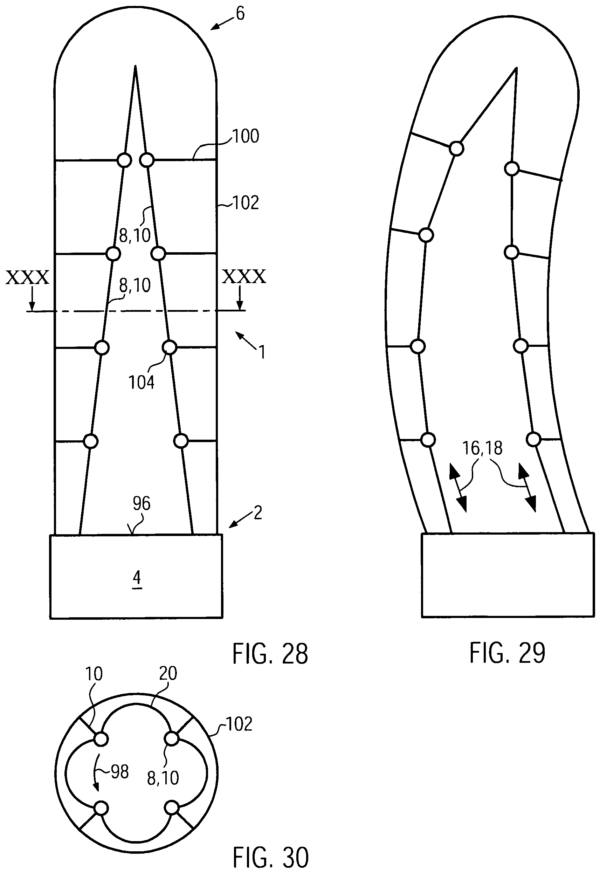

Eine weitere Ausführungsform, die eine dreidimensionale Krümmung des Manipulatorwerkzeuges 1 ermöglicht, ist in den Fig. 28 bis 30 dargestellt. Auch hier sind, über eine Grundfläche 96 der Werkzeugbasis 4 in einer Umfangsrichtung 98 verteilt, wenigstens drei, vorzugsweise aber vier Wangen 8, 10 vorgesehen, die in Umfangsrichtung, also quer zu ihrer Längserstreckung, durch Scharnierelemente 20 verbunden sind.

Über Abstandhalter 100 sind die Wangen 8, 10 mit einer flexiblen Hülle 102 verbunden, welche die Struktur nach außen abgrenzt und die Wangen 8, 10, die Scharnierelemente 20 sowie die Abstandhalter 100 aufnimmt. Diese Ausgestaltung erfordert nicht, dass alle Wangen 8, 10 biegesteif sind. Die Anzahl der mindestens notwendigen biegesteifen Wangen richtet sich nach der Anzahl der in dem Manipulatorwerkzeug 1 insgesamt vorhandenen Wangen und nach der zur Formgebung und Steifigkeit notwendigen Anzahl von biegesteifen Wangen.

Ist die Hülle 102 von ausreichender Steifigkeit, so dass sie die Wangen 8, 10 trägt, können diese auch segmentiert, d. h. durch Gelenke 104 gegliedert sein.

Die Abstandhalter 100 können an beliebiger Stelle zwischen den Wangen, Scharnierelementen oder Gelenken und der Hülle 102 verlaufen. Die Scharnierelemente 20 können im wesentlichen parallel zur Hülle verlaufen.

Wird in der bereits oben beschriebenen Weise in die Wangen 8, 10 eine Zug- und/oder Druckkraft 16, 18 eingeleitet, führt dies zu einer Verformung der Hülle 102, beispielsweise in der in Fig. 29 dargestellten Form.

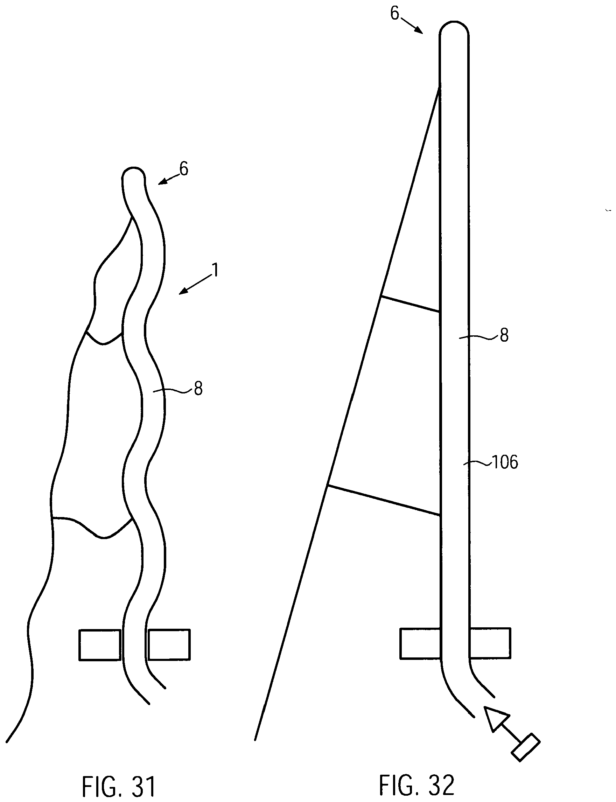

In der Ausführungsform der Fig. 31 ist die biegesteife Wange 8 hohl und flexibel, so dass sie durch ein Fluid 106 in ihrem Inneren aufgepumpt werden kann.

Bei dieser Ausgestaltung ist die Wange 8 biegeschlaff, wenn das Fluid 106 nicht unter Druck steht, bzw. entleert ist. Das Manipulatorwerkzeug 1 kann folglich durch sehr kleine Öffnungen geschoben werden und danach aufgepumpt werden.

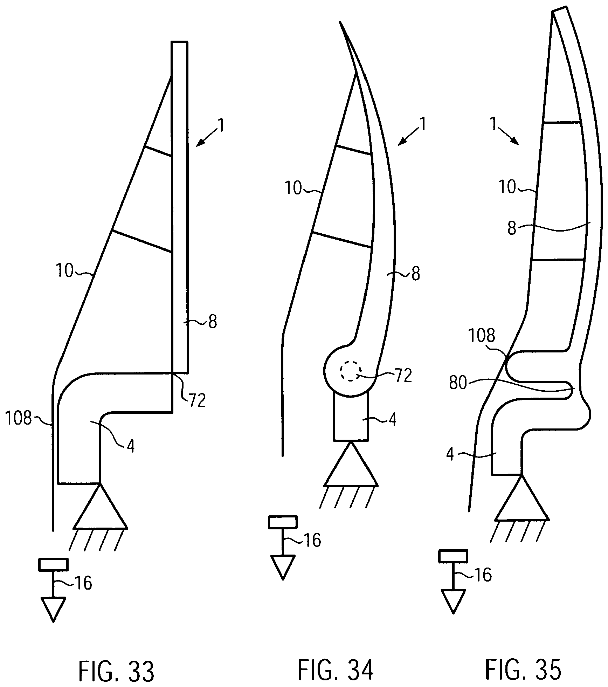

In den Ausführungsformen 1 der Fig. 33 bis 35 sind zum einen unterschiedliche Ausgestaltungen der Anbindung der wenigstens biegesteifen Wange 8 an die Werkzeugbasis 4 in Form von Gelenken 72 in Bereichen verringerter Steifigkeit 80 gezeigt. Gleichzeitig kann die Werkzeugbasis 4 ein Knie 108 aufweisen, das als Führung für die mit einer Zugkraft 16 beaufschlagbaren, wenigstens zugfeste Wange 10 dient.

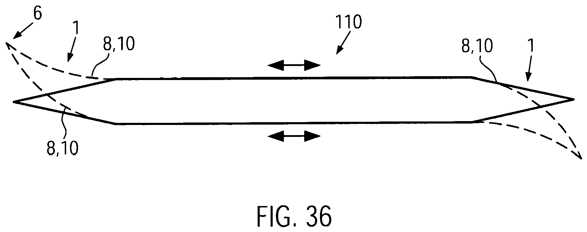

Fig. 36 zeigt eine Ausgestaltung, bei der an den Enden eines längsgestreckten Werkzeuges 110 zwei Manipulatorwerkzeuge 1 angeordnet sind, deren Wangen 8, 10 miteinander verbunden sind, so dass sie stets gleichzeitig und gegenläufig betätigt werden.

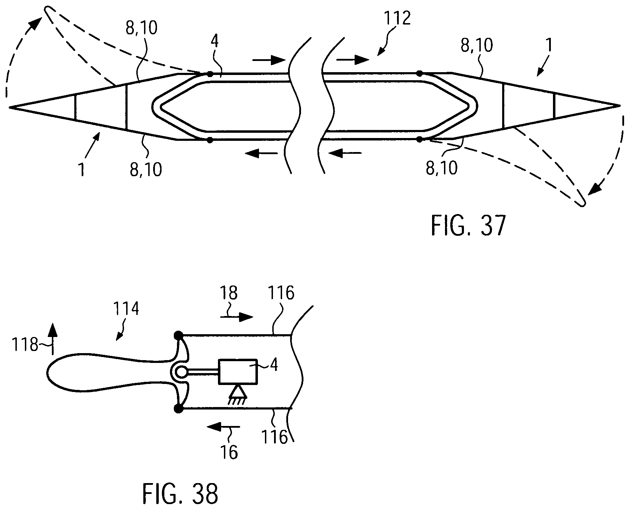

Fig. 37 zeigt beispielhaft einen Schnitt durch eine solche Ausgestaltung. Die Werkzeugbasis 4 wird von einem beispielsweise polygonalen Grundkörper gebildet, an dessen beiden gegenüberliegenden Enden die Manipulatorwerkzeuge 1 angeordnet sind. Der Bereich 112 zwischen den beiden Manipulatorwerkzeugen kann als Griff ausgestaltet sein. Wird der Werkzeugbasis 4 eine Scherbewegung als Arbeitsbewegung aufgeprägt, wird entsprechend an einer Seite der Werkzeugbasis gleichzeitig eine Zugkraft bzw. Längsbewegung in die eine Wange des einen Manipulatorwerkzeuges und eine Druckkraft bzw. Längsbewegung in gleicher Richtung in die Wange des anderen Manipulatorwerkzeuges eingeleitet. An der anderen Seite herrscht eine umgekehrte Kraftrichtung. Daraus resultiert eine Verformung der Manipulatorwerkzeuge 1 in der in Fig. 37 gestrichelt dargestellten Form.

Fig. 38 zeigt eine Handhabe 114 zur gleichzeitigen Erzeugung einer Druckkraft 18 und einer Zugkraft 16 in einem nicht dargestellten Manipulatorwerkzeug 1, das mit dem Zug-/Druckmitteln 116 verbunden ist. Die Handhabe 14 ist gelenkig gelagert und kann, wie durch den Pfeil 118 angedeutet ist, verkippt werden. An dem in Kipprichtung gelegenen Zug-/Druckmittel wird eine Druckkraft, der von der Kipprichtung abgewandten Seite ein Zugkraft 16 erzeugt.

Die Lagerung der Handhabe 108 erfolgt vorzugsweise an einer Stelle, die mit der Werkzeugbasis 4 starr verbunden ist.

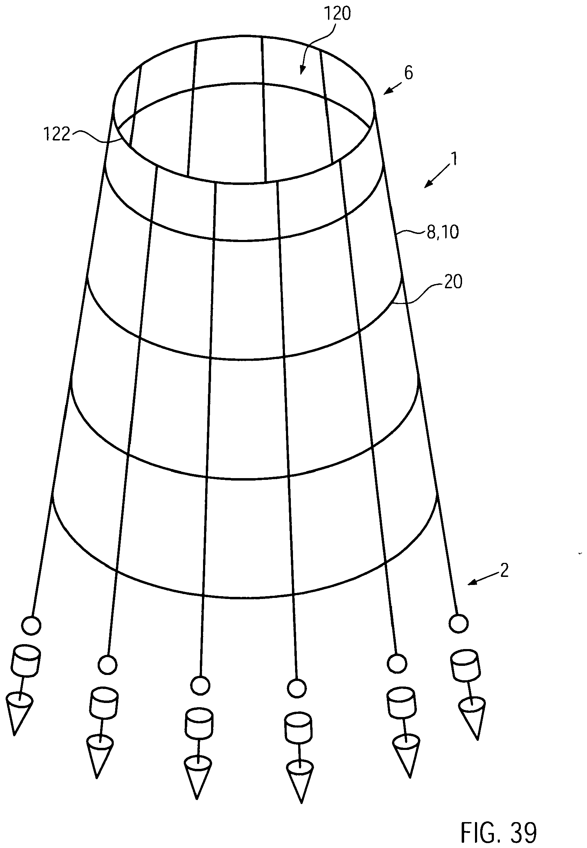

Fig. 39 zeigt die Ausgestaltung eines Manipulatorwerkzeuges 1 in Schlauchform. Eine Vielzahl von Wangen 8, 10, die wenigstens teilweise unabhängig voneinander längsverschieblich und teilweise an der Werkzeugbasis festgelegt sein können, sind ringförmig um ein Zentrum angeordnet und jeweils mit der benachbarten Wange durch Scharnierelemente 20 verbunden. Aus dieser Anordnung ergibt sich ein Innenraum 120, der von den Scharnierelementen ringförmig und von den Wangen 8, 10 in Längsrichtung begrenzt ist.

Damit sich der Innenraum 120 zumindest am proximalen Ende 2 frei öffnet und somit zur Aufnahme von weiteren Instrumenten oder zur Durchleitung von Fluiden eignet, sind benachbarte Wangen 8, 10 an ihrem distalen Ende 6 nicht keilförmig, sondern stumpf durch ein jeweiliges Abschlusselement 122 verbunden. Die Verwendung eines solchen Abschlusselements 122 ist für die erfindungsgemäße Wirkung unerheblich, so lange wie bei der keilförmigen Verbindungsstelle 14 (Fig. 1) sichergestellt ist, dass die Winkel zwischen den benachbarten Wangen 8, 10, die an dieser Stelle miteinander verbunden sind, sich bei der Verformung nicht oder nur unwesentlich ändern.

Anstelle der Betätigung einzelner längsverschieblicher Wangen können die beweglichen Wangen einer Weiterbildung am proximalen Ende durch einen in sich steifen Ring verbunden sein, über dessen Bewegung insbesondere Verkippung die Verformung des Schlauches bewirkt wird, indem die daran befestigten Wangen gleichzeitig verschoben werden. In dieser Ausgestaltung kann der Schlauch als separates Instrument beispielsweise als beweglicher Tubus verwendet werden.

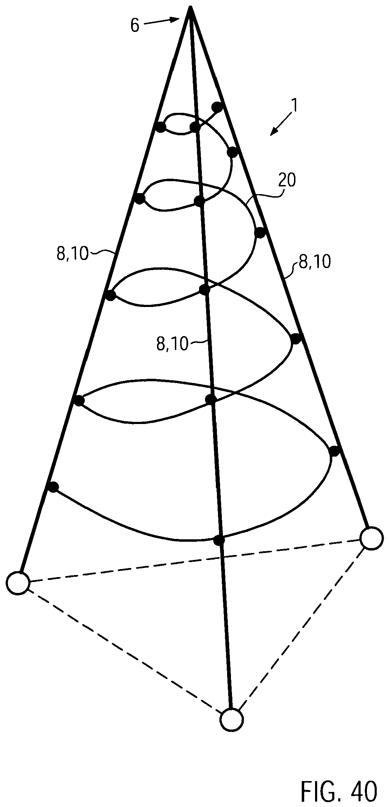

Die Form der Scharnierelemente 20 ist, solange sie ihre mit Bezug auf die Fig. 2 beschriebene Funktion erfüllen, in erster Linie durch den Anwendungsfall des Manipulatorwerkzeuges 1 bestimmt. So kann beispielsweise ein durchgängiges Scharnierelement sich wendelförmig zwischen mehreren Wangen 8, 10 erstrecken, wie Fig. 40 zeigt. Anstelle einer Wendel können Kreise, wie in Fig. 39 oder andere geometrische Ausgestaltungen zum Einsatz kommen.

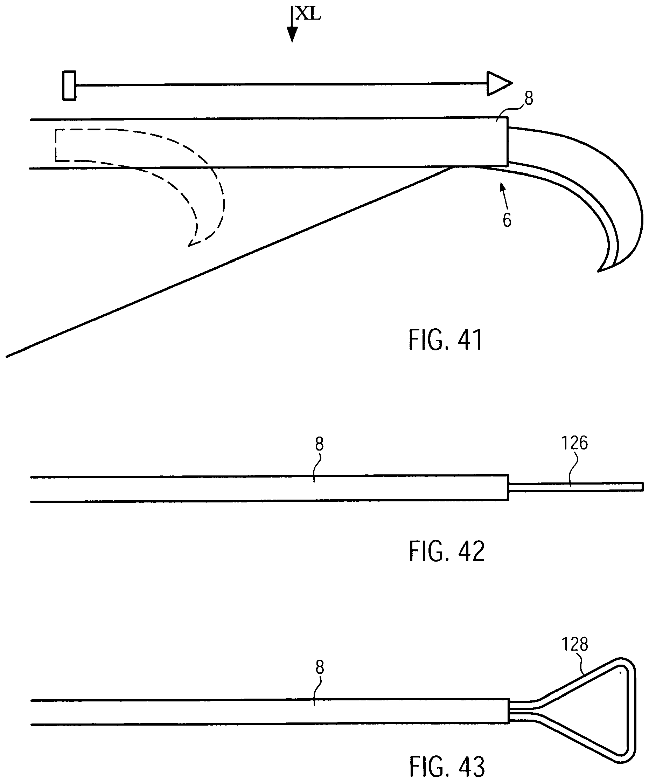

Schließlich kann die wenigstens biegesteife Wange 8 selbst hohl sein und als Werkzeugaufnahme für darin längsverschieblich aufgenommene Werkzeuge dienen. Dies ist in den Fig. 41 bis 43 dargestellt. So können Werkzeuge, wie eine Klinge 124, eine Nadel 126 oder eine Schlaufe 128 in der biegesteifen Wange 8 aufgenommen sein. Selbstverständlich können auch Fluide durchgeleitet werden oder elektrische bzw. optische Geräte aufgenommen sein.

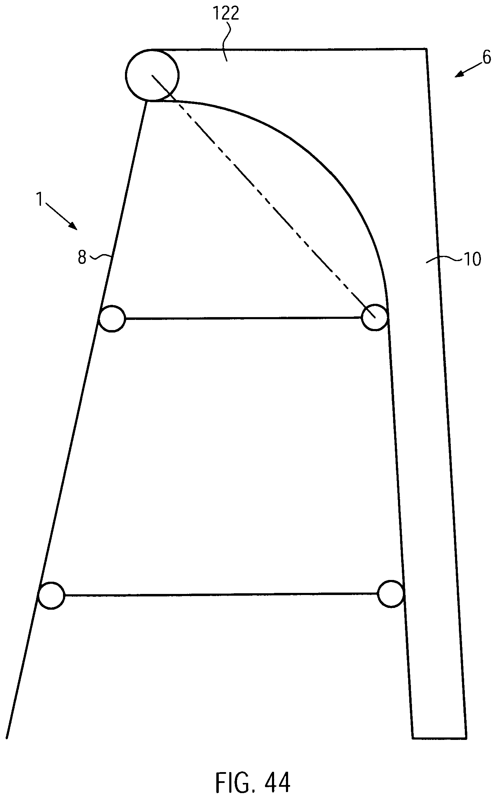

Fig. 44 schließlich zeigt, dass am distalen Ende 6 die Verbindung zwischen den Wangen 8, 10 nicht keilförmig sein muss, sondern auch stumpf durch das Abschlusselement 122, das beispielsweise einstückig mit der wenigstens biegesteifen Wange 10 ausgebildet sein, erfolgt. Wesentlich ist lediglich, dass sich am distalen Ende 6 eine im Wesentlichen über die Verformung des Manipulatorwerkzeuges 1 winkelsteife Dreieckfigur ergibt, die in Fig. 44 durch die strichpunktierte Linie angedeutet ist.

### Bezugszeichenliste:

- 1: Manipulatorwerkzeug
- 2: proximales Ende
- 4: Werkzeugbasis
- 6: distales Ende
- 8: wenigstens biegesteife bzw. biegesteife Wange
- 10: wenigstens zugfeste Wange
- 12: Manipulatorebene
- 14: Verbindungsstelle von 8 und 10
- 16: Zugkraft
- 18: Druckkraft
- 20: Scharnierelement
- 22: Bewegung von 6
- 24: Verankerungspunkt von 20 an 10
- 26: Länge von 20
- 28: Kreisbahn von 24 um 30
- 30: Verankerungspunkt von 20 an 8
- 32: Kreisbahn von 30 und 24
- 34: Winkel zwischen 8 und 20
- 36: Winkel zwischen 10 und 20
- 38: Druckkraft
- 40: Linearführung
- 42: Linearführung
- 44: Werkzeughalter
- 46: Werkzeug
- 48: Fortsatz
- 50: Abwandlung von 1
- 52: wenigstens biegesteife Wange von 50
- 54: wenigstens zugfeste Wange von 50
- 58, 60: Enden von 52
- 62: Druckkraft
- 64: Bewegung
- 66: Halte- und/oder Spreizwerkzeug
- 68,70: Gegenstand
- 72: Gelenk
- 74: Umlenkrolle
- 76: Zwischenraum
- 78: Zugmittel
- 80: Bereich verringerter Steifigkeit
- 82: Breite von 66
- 83: gelenkige Verbindung
- 84: Wurzel
- 86: Stamm
- 87: Backe
- 88: Kupplung
- 90: Greifbereich
- 91: Öffnung in 4
- 94: Backe
- 96: Grundfläche von 4
- 98: Umfangsrichtung
- 100: Abstandhalter
- 102: Hülle
- 104: Gelenke
- 106: Fluid
- 108: Knie
- 110: Werkzeug
- 112: Griff
- 114: Handhabe
- 116: Zug-/Druckmittel
- 118: Kipprichtung
- 120: Innenraum
- 122: Abschlusselement
- 124: Klinge
- 126: Nadel

## Patentansprüche

1. Halte- und/oder Spreizwerkzeug mit wenigstens zwei bezüglich eines Zwischenraumes (76) einander gegenüberliegenden Backen, von denen wenigstens eine Backe bezüglich der anderen Backe beweglich ist, wobei wenigstens zwei bezüglich eines Zwischenraumes (76) einander gegenüberliegende Backen gebildet sind von einem biegsamen Manipulatorwerkzeug (1) mit einem gegenüber einem proximalen Ende (2) in wenigstens einer Manipulationsebene (12) beweglichen distalen Ende (6) mit wenigstens zwei nebeneinander beabstandet verlaufenden, wenigstens in der Manipulationsebene flexiblen und sich vom proximalen Ende zum distalen Ende erstreckenden Wangen (8, 10), die zwischen dem proximalen Ende und dem distalen Ende durch wenigstens ein wenigstens zugfestes Scharnierelement (20) gegeneinander scherbeweglich verbunden und am proximalen Ende voneinander beabstandet gehalten sind, wobei die eine Wange (8) wenigstens biegesteif und die andere Wange (10) wenigstens zugfest ausgestaltet und die wenigstens zugfeste Wange an ihrem distalen Ende mit der wenigstens biegesteifen Wange Zugkraft übertragend verbunden ist, **dadurch gekennzeichnet, dass** wenigstens eine Wange (8, 10) am proximalen Ende (2) in ihrer Längsrichtung antreibbar ausgestaltet ist, wobei die einander zugewandten, an den Zwischenraum (76) angrenzenden wenigstens biegesteifen Wangen (8) oder die einander zugewandten, an den Zwischenraum (76) angrenzenden wenigstens zugfesten Wangen (10) der wenigstens zwei Manipulatorwerkzeuge (1) am proximalen Ende (2) zusammengeführt sind.

2. Halte- und/oder Spreizwerkzeug (66) nach Anspruch 1, **dadurch gekennzeichnet, dass** das Manipulatorwerkzeug (1) an seinem distalen Ende (6) mit dem distalen Ende der anderen Backe (1) gelenkig verbunden ist.

3. Halte- und/oder Spreizwerkzeug nach Anspruch 1 oder 2, **dadurch gekennzeichnet, dass** die einander zugewandten, an den Zwischenraum (76) angrenzenden wenigstens biegesteifen Wangen (8) oder die einander zugewandten, an den Zwischenraum (76) angrenzenden wenigstens zugfesten Wangen (10) der wenigstens zwei Manipulatorwerkzeuge (1) am proximalen Ende (2) verbunden sind.

4. Halte- und/oder Spreizwerkzeug nach einem der Ansprüche 1 bis 3, **dadurch gekennzeichnet, dass** es aus einem integralen Körper einstückig geformt ist.

5. Halte- und/oder Spreizwerkzeug nach einem der Ansprüche 1 bis 4, **dadurch gekennzeichnet, dass** die wenigstens zugfesten Wangen (10) an den Zwischenraum (76) angrenzen.

6. Halte- und/oder Spreizwerkzeug (66) nach Anspruch 1, wobei wenigstens ein Manipulatorwerkzeug (1) aus Anspruch 1 **dadurch gekennzeichnet ist, dass** die antreibbare Wange (8, 10) zug- und biegesteif ausgebildet ist.

7. Halte- und/oder Spreizwerkzeug (66) nach Anspruch 1, wobei wenigstens ein Manipulatorwerkzeug (1) aus Anspruch 1 oder 6 **dadurch gekennzeichnet ist, dass** am distalen Ende (6) ein Werkzeughalter (44) angeordnet ist.

8. Halte- und/oder Spreizwerkzeug (66) nach Anspruch 1, wobei wenigstens ein Manipulatorwerkzeug (1) aus einem der Ansprüche 1, 6 und 7 **dadurch gekennzeichnet ist, dass** das wenigstens eine Scharnierelement (20) an einer Werkzeugbasis (4) befestigt ist.

9. Halte- und/oder Spreizwerkzeug (66) nach Anspruch 1, wobei wenigstens ein Manipulatorwerkzeug (1) aus einem der Ansprüche 1, 6 bis 8 **dadurch gekennzeichnet ist, dass** wenigstens eine biegesteife Wange (8) eine in Richtung vom proximalen Ende (2) zum distalen Ende (6) sich verändernde Steifigkeit aufweist.

10. Halte- und/oder Spreizwerkzeug (66) nach Anspruch 1, wobei wenigstens ein Manipulatorwerkzeug (1) aus einem der Ansprüche 1, 6 bis 9 **dadurch gekennzeichnet ist, dass** am distalen Ende (6) die eine Wange (8) über die andere Wange (10) hinaus unter Bildung eines Fortsatzes (48) verlängert ist.

11. Halte- und/oder Spreizwerkzeug (66) nach Anspruch 1, wobei wenigstens ein Manipulatorwerkzeug (1) aus einem der Ansprüche 1, 6 bis 10 **dadurch gekennzeichnet ist, dass** die eine Wange (8, 10) am proximalen Ende (2) gelenkig gelagert ist.

12. Halte- und/oder Spreizwerkzeug (66) nach Anspruch 1, wobei wenigstens ein Manipulatorwerkzeug (1) aus einem der Ansprüche 1, 6 bis 11 **dadurch gekennzeichnet ist, dass** die wenigstens biegesteife Wange (8) als Federelement ausgestaltet ist, dessen Federkraft im verformten Zustand in die wenigstens zugfeste Wange (10) eingeleitet ist.

13. Halte- und/oder Spreizwerkzeug (66) nach Anspruch 1, wobei wenigstens ein Manipulatorwerkzeug (1) aus einem der Ansprüche 1, 6 bis 12 **dadurch gekennzeichnet ist, dass** wenigstens drei Wangen (8, 10) vorgesehen sind, von denen wenigstens zwei längsbeweglich angetrieben sind.

14. Halte- und/oder Spreizwerkzeug (66) nach Anspruch 1, wobei wenigstens ein Manipulatorwerkzeug (1) aus Anspruch 13 **dadurch gekennzeichnet ist, dass** die wenigstens drei Wangen (8, 10) in einer Manipulatorebene (12) liegen.

15. Halte- und/oder Spreizwerkzeug (66) nach Anspruch 1, wobei wenigstens ein Manipulatorwerkzeug (1) aus Anspruch 13 **dadurch gekennzeichnet ist, dass** die wenigstens drei Wangen (8, 10) in unterschiedlichen Manipulatorebenen (12) liegen.

16. Halte- und/oder Spreizwerkzeug (66) nach Anspruch 1, wobei wenigstens ein Manipulatorwerkzeug (1) aus Anspruch 14 oder 15 **dadurch gekennzeichnet ist, dass** jeweils benachbarte Wangen (8, 10) durch Scharnierelemente (20) verbunden sind.

17. Halte- und/oder Spreizwerkzeug (66) nach Anspruch 1, wobei wenigstens ein Manipulatorwerkzeug (1) aus einem der Ansprüche 13 bis 16 **dadurch gekennzeichnet ist, dass** die Wangen (8, 10) um einen Innenraum herum angeordnet sind.

18. Halte- und/oder Spreizwerkzeug (66) nach Anspruch 1, wobei wenigstens ein Manipulatorwerkzeug (1) aus Anspruch 17 **dadurch gekennzeichnet ist, dass** die Scharnierelemente (20) um den Innenraum herum angeordnet sind.

19. Halte- und/oder Spreizwerkzeug (66) nach Anspruch 1, wobei wenigstens ein Manipulatorwerkzeug (1) aus Anspruch 17 oder 18 **dadurch gekennzeichnet ist, dass** der Innenraum zum distalen Ende (6) hin offen ist.

20. Halte- und/oder Spreizwerkzeug (66) nach Anspruch 1, wobei wenigstens ein Manipulatorwerkzeug (1) aus einem der Ansprüche 1, 6 bis 19 **dadurch gekennzeichnet ist, dass** die wenigstens biegesteife Wange (8) am distalen Ende (6) mit jeweils wenigstens zwei wenigstens zugfesten Wangen (10) verbunden ist.

21. Halte- und/oder Spreizwerkzeug (66) nach Anspruch 1, wobei wenigstens ein Manipulatorwerkzeug (1) aus einem der Ansprüche 1, 6 bis 20 **dadurch gekennzeichnet ist, dass** die wenigstens biegesteife Wange 8 hohl ist.

22. Halte- und/oder Spreizwerkzeug nach eine der Ansprüche 1 bis 21, **dadurch gekennzeichnet, dass** ein Paar sich bezüglich des Zwischenraums (76) gegenüberliegender Backen als Manipulatorwerkzeug (1) aus einem der Ansprüche 1 und 6 bis 21 ausgebildet sind.

## Claims

1. Holding and/or expanding tool with at least two jaws opposite with respect to a space (76), of which at least one jaw is movable with respect to the other jaw, wherein at least two jaws, which are adjacent to the space (76), are formed by a flexible manipulator tool (1) with a distal end (6) movable in at least one manipulation plane (12) with respect to a proximal end (2), with at least two cheeks (8, 10) extending side by side to and spaced apart from each other, which are flexible at least in the manipulation plane and extend from the proximal end to the distal end, the cheeks (8, 10) being connected between the proximal end and the distal end by at least one at least tension-proof hinge element (20) to permit a shearing movement relative to each other, and being held at the proximal end at a distance from each other, wherein the one cheek (8) is configured to be at least flexurally stiff and the other cheek (10) to be at least tension-proof, and the at least tension-proof cheek is connected at its distal end to the at least flexurally stiff cheek so as to transmit a pulling force, **characterized in that** one cheek (8, 10) is embodied to be driven at the proximal end (2) in the longitudinal direction, wherein the at least tension-proof cheeks (8) of the at least two manipulator tools (1), which face each other and are adjacent to the space (76), or the at least flexurally stiff cheeks (20) of the at least two manipulator tools (1), which face each other and are adjacent to the space (76), are brought together at the proximal end (2).

2. Holding and/or expanding tool (66) according to claim 1, **characterized in that** the manipulator tool (1) is hinged at its distal end (6) to the distal end of the other jaw (1).

3. Holding and/or expanding tool according to claim 1 or 2, **characterized in that** the at least tension-proof cheeks (8) of the at least two manipulator tools (1), which face each other and are adjacent to the space (76), or the at least flexurally stiff cheeks (20) of the at least two manipulator tools (1), which face each other and are adjacent to the space (76), are connected to each other at the proximal end (2).

4. Holding and/or expanding tool according to one of claims 1 to 3, **characterized in that** it is formed of an integral body in one piece.

5. Holding and/or expanding tool according to one of claims 1 to 4, **characterized in that** the at least tension-proof cheeks (10) are adjacent to the space (76).

6. Holding and/or expanding tool (66) according to claim 1, wherein at least one manipulator tool (1) of claim 1 is **characterized in that** the drivable cheek (8, 10) is configured to be tension-proof and flexurally stiff.

7. Holding and/or expanding tool (66) according to claim 1, wherein at least one manipulator tool (1) of 1 or 6 is **characterized in that** a tool holder (44) is arranged at the distal end (6).

8. Holding and/or expanding tool (66) according to claim 1, wherein at least one manipulator tool (1) of one of claims 1, 6 and 7 is **characterized in that** the at least one hinge element (20) is fixed to a tool base (4).

9. Holding and/or expanding tool (66) according to claim 1, wherein at least one manipulator tool (1) according to one of claims 1 and 6 to 8 is **characterized in that** at least one flexurally stiff cheek (8) comprises a stiffness changing in the direction from the proximal end (2) to the distal end (6).

10. Holding and/or expanding tool (66) according to claim 1, wherein at least one manipulator tool (1) of one of claims 1 and 6 to 9 is **characterized in that** the one cheek (8) is elongated beyond the other cheek (10) at the distal end (6), forming an extension (48).

11. Holding and/or expanding tool (66) according to claim 1, wherein at least one manipulator tool (1) of one of claims 1 and 6 to 10 is **characterized in that** the one cheek (8, 10) is supported in an articulated manner at the proximal end (2).

12. Holding and/or expanding tool (66) according to claim 1, wherein at least one manipulator tool (1) of one of claims 1 and 6 to 11 is **characterized in that** the at least flexurally stiff cheek (8) is embodied as spring element of which the spring force is introduced into the at least tension-proof cheek (10) in a deformed state.

13. Holding and/or expanding tool (66) according to claim 1, wherein at least one manipulator tool (1) of one of claims 1 and 6 to 12 is **characterized in that** at least three cheeks (8, 10) are provided, of which at least two are driven to move lengthwise.

14. Holding and/or expanding tool (66) according to claim 1, wherein at least one manipulator tool (1) of claim 13 is **characterized in that** the at least three cheeks (8, 10) are located in one manipulator plane (12).

15. Holding and/or expanding tool (66) according to claim 1, wherein at least one manipulator tool (1) of claim 13 is **characterized in that** the at least three cheeks (8, 10) are located in different manipulator planes (12).

16. Holding and/or expanding tool (66) according to claim 1, wherein at least one manipulator tool (1) of claim 14 or 15 is **characterized in that** adjacent cheeks (8, 10) are each connected by hinge elements (20).

17. Holding and/or expanding tool (66) according to claim 1, wherein at least one manipulator tool (1) according to one of claims 13 to 16, **characterized in that** the cheeks (8, 10) are arranged around an interior.

18. Holding and/or expanding tool (66) according to claim 1, wherein at least one manipulator tool (1) of claim 17 is **characterized in that** the hinge elements (20) are arranged around the interior.

19. Holding and/or expanding tool (66) according to claim 1, wherein at least one manipulator tool (1) of claim 17 or 18 is **characterized in that** the interior is open towards the distal end (6).

20. Holding and/or expanding tool (66) according to claim 1, wherein at least one manipulator tool (1) of one of claims 1 and 6 to 19 is **characterized in that** the at least flexural stiff cheek (8) is connected with at least two at least tension-proof cheeks (10) at the distal end respectively.

21. Holding and/or expanding tool (66) according to claim 1, wherein at least one manipulator tool (1) of one of claims 1 and 6 to 20 is **characterized in that** the at least flexurally stiff cheek (8) is hollow.

22. Holding and/or expanding tool (66) according to any of the claims 1 to 21 is **characterized in that** a pair jaws opposite with respect to the space (76) is embodied as manipulator tool (1) according to one of claims 1 and 6 to 21.

## Revendications

1. Outil de maintien, et/ou d'écartement comprenant au moins deux mâchoires mutuellement opposées par rapport à un espace intermédiaire (76) et dont au moins une mâchoire est mobile par rapport à l'autre mâchoire, outil
dans lequel au moins deux mâchoires mutuellement opposées par rapport à un espace intermédiaire (76) sont formées par un outil de manipulation (1) flexible, avec une extrémité distale (6) mobile par rapport à une extrémité proximale (12) dans au moins un plan de manipulation (12), et avec au moins deux joues (8, 10), qui s'étendent côte à côte à distance l'une de l'autre, sont flexibles au moins dans le plan de manipulation et s'étendent de l'extrémité proximale vers l'extrémité distale, et qui, entre l'extrémité proximale et l'extrémité distale, sont reliées de façon mobile l'une par rapport à l'autre à la manière d'un mouvement de cisaillement par l'intermédiaire d'au moins un élément de charnière (20) au moins résistant à la traction, et sont maintenues à distance l'une de l'autre au niveau de l'extrémité proximale, et
dans lequel une joue (8) est d'une configuration au moins rigide en flexion et l'autre joue (10) d'une configuration au moins résistante à la traction, et ladite joue au moins résistante à la traction est reliée, à son extrémité distale, à la joue au moins rigide en flexion, en permettant la transmission d'une force de traction,
**caractérisé en ce qu'**au moins une joue (8, 10) est, à l'extrémité proximale (2), réalisée de manière à pouvoir être entraînée dans sa direction longitudinale, les joues (8) au moins rigides en flexion dirigées l'une vers l'autre et adjacentes à l'espace intermédiaire (76) ou bien les joues (10) au moins résistantes en traction dirigées l'une vers l'autre et adjacentes à l'espace intermédiaire (76), desdits au moins deux outils de manipulation (1), sont réunies à l'extrémité proximale (2).

2. Outil de maintien et/ou d'écartement (66) selon la revendication 1, **caractérisé en ce que** l'outil de manipulation (1) est, à son extrémité distale (6), relié de manière articulée à l'extrémité distale de l'autre mâchoire (1).

3. Outil de maintien et/ou d'écartement selon la revendication 1 ou la revendication 2, **caractérisé en ce que** les joues (8) au moins rigides en flexion dirigées l'une vers l'autre et adjacentes à l'espace intermédiaire (76), ou bien les joues (10) au moins résistantes en traction dirigées l'une vers l'autre et adjacentes à l'espace intermédiaire (76), desdits au moins deux outils de manipulation (1), sont reliées mutuellement à l'extrémité proximale (2).

4. Outil de maintien et/ou d'écartement selon l'une des revendications 1 à 3, **caractérisé en ce qu'**il est réalisé par un corps intégral d'un seul tenant.

5. Outil de maintien et/ou d'écartement selon l'une des revendications 1 à 4, **caractérisé en ce que** les joues (10) au moins résistantes à la traction sont adjacentes à l'espace intermédiaire (76).

6. Outil de maintien et/ou d'écartement (66) selon la revendication 1, dans lequel au moins un outil de manipulation (1) de la revendication 1 est **caractérisé en ce que** la joue (8, 10) pouvant être entraînée est réalisée rigide en flexion et en traction.

7. Outil de maintien et/ou d'écartement (66) selon la revendication 1, dans lequel au moins un outil de manipulation (1) de la revendication 1 ou de la revendication 6 est **caractérisé en ce qu'**à l'extrémité distale (6) est agencé un porte-outil (44).

8. Outil de maintien et/ou d'écartement (66) selon la revendication 1, dans lequel au moins un outil de manipulation (1) de l'une des revendications 1, 6 et 7 est **caractérisé en ce que** ledit au moins un élément de charnière (20) est fixé au niveau d'une base d'outil (4).

9. Outil de maintien et/ou d'écartement (66) selon la revendication 1, dans lequel au moins un outil de manipulation (1) de l'une des revendications 1, 6 à 8 est **caractérisé en ce qu'**au moins une joue (8) rigide en flexion présente une rigidité variant dans la direction de l'extrémité proximale (2) vers l'extrémité distale (6).

10. Outil de maintien et/ou d'écartement (66) selon la revendication 1, dans lequel au moins un outil de manipulation (1) de l'une des revendications 1, 6 à 9 est **caractérisé en ce qu'**à l'extrémité distale (6) une joue (8) est prolongée au-delà de l'autre joue (10) en formant un appendice de prolongement (48).

11. Outil de maintien et/ou d'écartement (66) selon la revendication 1, dans lequel au moins un outil de manipulation (1) de l'une des revendications 1, 6 à 10 est **caractérisé en ce qu'**une joue (8, 10) est montée de manière articulée à l'extrémité proximale (2).

12. Outil de maintien et/ou d'écartement (66) selon la revendication 1, dans lequel au moins un outil de manipulation (1) de l'une des revendications 1, 6 à 11 est **caractérisé en ce que** ladite joue (8) au moins rigide en flexion est réalisée en tant qu'élément de ressort dont la force de ressort est, dans l'état déformé, introduite dans la joue (10) au moins résistante à la traction.

13. Outil de maintien et/ou d'écartement (66) selon la revendication 1, dans lequel au moins un outil de manipulation (1) de l'une des revendications 1, 6 à 12 est **caractérisé en ce que** sont prévues au moins trois joues (8, 10), dont au moins deux sont entraînées selon un mouvement longitudinal.

14. Outil de maintien et/ou d'écartement (66) selon la revendication 1, dans lequel au moins un outil de manipulation (1) de la revendication 13 est **caractérisé en ce que** lesdites au moins trois joues (8, 10) se situent dans un plan de manipulation (12).

15. Outil de maintien et/ou d'écartement (66) selon la revendication 1, dans lequel au moins un outil de manipulation (1) de la revendication 13 est **caractérisé en ce que** lesdites au moins trois joues (8, 10) se situent dans des plans de manipulation (12) différents.

16. Outil de maintien et/ou d'écartement (66) selon la revendication 1, dans lequel au moins un outil de manipulation (1) de la revendication 14 ou de la revendication 15 est **caractérisé en ce que** des joues (8, 10) respectivement voisines sont reliées par des éléments de charnière (20).

17. Outil de maintien et/ou d'écartement (66) selon la revendication 1, dans lequel au moins un outil de manipulation (1) de l'une des revendications 13 à 16 est **caractérisé en ce que** les joues (8, 10) sont agencées tout autour d'un espace intérieur.

18. Outil de maintien et/ou d'écartement (66) selon la revendication 1, dans lequel au moins un outil de manipulation (1) de la revendication 17 est **caractérisé en ce que** les éléments de charnière (20) sont agencés tout autour de l'espace intérieur.

19. Outil de maintien et/ou d'écartement (66) selon la revendication 1, dans lequel au moins un outil de manipulation (1) de la revendication 17 ou de la revendication 18 est **caractérisé en ce que** l'espace intérieur est ouvert en direction de l'extrémité distale (6).

20. Outil de maintien et/ou d'écartement (66) selon la revendication 1, dans lequel au moins un outil de manipulation (1) de l'une des revendications 1, 6 à 19 est **caractérisé en ce que** la joue (8) au moins rigide en flexion est, au niveau de l'extrémité distale (6), reliée à respectivement au moins deux joues (10) au moins résistantes à la traction.

21. Outil de maintien et/ou d'écartement (66) selon la revendication 1, dans lequel au moins un outil de manipulation (1) de l'une des revendications 1, 6 à 20 est **caractérisé en ce que** la joue (8) au moins rigide en flexion est creuse.

22. Outil de maintien et/ou d'écartement selon l'une des revendications 1 à 21, **caractérisé en ce qu'**une paire de mâchoires mutuellement opposées par rapport à l'espace intermédiaire (76), sont réalisées en tant qu'outil de manipulation (1) de l'une des revendications 1 et 6 à 21.
